# EUROPEAN PATENT APPLICATION

(11) **EP 2 103 685 A1**
(43) Date of publication of application: **23.09.2009**
(21) Application number: 08005378.8
(22) Date of filing: 20.03.2008
(51) Int. Cl.: C12N 5/06, C12N 15/09

(54) **Vectors and methods for generating vector-free induced pluripotent stem (iPS) cells using site-specific recombination**

(71) Applicant: Helmholtz Zentrum München Deutsches Forschungszentrum für Gesundheit und Umwelt (GmbH), 85764 Neuherberg (DE)
(72) Inventor: The designation of the inventor has not yet been filed
(74) Representative: Vossius & Partner

(57) **Abstract**

The present invention relates to a DNA molecule comprising: (a) a first DNA sequence comprising: (aa) a coding sequence giving rise upon transcription to a factor that contributes to the reprogramming of a somatic cell into an induced pluripotent stem (iPS) cell; (ab) a promoter mediating the transcription of said coding sequence; and (ac) two sequence motifs that mediate excision of (aa) and/or (ab) from the DNA molecule, wherein one sequence motif is positioned 5' and the other sequence motif is positioned 3' of the sequence to be excised; (b) a second DNA sequence comprising a sequence motif that mediates site-specific integration of (a) into another DNA molecule. Further, the invention relates to DNA molecule comprising: (a) a first DNA sequence comprising: (aa) a coding sequence giving rise upon transcription to a factor that contributes to the reprogramming of a somatic cell into an induced pluripotent stem cell; and (ab) a promoter mediating the transcription of said coding sequence; (b) a second DNA sequence comprising: (ba) a sequence motif that mediates extrachromosomal self-replication of the DNA-molecule; and (bb) two sequence motifs that mediate excision of at least said sequence motif of (ba) from the second DNA sequence (b), wherein one sequence motif is located 5' of (ba) and the other sequence motif 3' of (ba). Also, the invention relates to a vector comprising the DNA molecule of the invention, a method for assembly of said vector and a somatic cell comprising said DNA molecule or said vector vector of the invention. Furthermore, the invention relates to methods to generate an induced pluripotent stem (iPS) cell, an induced pluripotent stem cell obtainable by said methods, to a kit comprising the DNA molecule of the invention, to a cell line or cell culture collection comprising the induced pluripotent stem cell of the invention, to the use of said cell or cell line as a research tool, to a method to generate a transgenic non-human animal and to a non-human animal generated by said method. Finally, the invention relates to a composition for gene therapy, regenerative medicine, cell therapy or drug screening.

## Description

The present invention relates to a DNA molecule comprising: (a) a first DNA sequence comprising: (aa) a coding sequence giving rise upon transcription to a factor that contributes to the reprogramming of a somatic cell into an induced pluripotent stem (iPS) cell; (ab) a promoter mediating the transcription of said coding sequence; and (ac) two sequence motifs that mediate excision of (aa) and/or (ab) from the DNA molecule, wherein one sequence motif is positioned 5' and the other sequence motif is positioned 3' of the sequence to be excised; (b) a second DNA sequence comprising a sequence motif that mediates site-specific integration of (a) into another DNA molecule. Further, the invention relates to a DNA molecule comprising: (a) a first DNA sequence comprising: (aa) a coding sequence giving rise upon transcription to a factor that contributes to the reprogramming of a somatic cell into an induced pluripotent stem cell; and (ab) a promoter mediating the transcription of said coding sequence; (b) a second DNA sequence comprising: (ba) a sequence motif that mediates extrachromosomal self-replication of the DNA-molecule; and two sequence motifs that mediate excision of at least said sequence motif of (ba) from the second DNA sequence (b), wherein one sequence motif is located 5' of (ba) and the other sequence motif 3' of (ba). Also, the invention relates to a vector comprising the DNA molecule of the invention, a method for assembly of said vector and a somatic cell comprising said DNA molecule or said vector of the invention. Furthermore, the invention relates to methods to generate an induced pluripotent stem (iPS) cell, an induced pluripotent stem cell obtainable by said methods, to a kit comprising the DNA molecule of the invention, to a cell line or cell culture collection comprising the induced pluripotent stem cell of the invention, to the use of said cell or cell line as a research tool, to a method to generate a transgenic non-human animal and to a non-human animal generated by said method. Finally, the invention relates to a composition for gene therapy, regenerative medicine, cell therapy or drug screening.

Several documents are cited throughout the text of this specification. The disclosure content of the documents cited herein (including manufacturer's specifications, instructions, etc.) is herewith incorporated by reference.

Pluripotent stem cells like embryonic stem (ES) cells are hallmarked by their ability to self-renew and differentiate into a wide variety of cell types. ES cells can be differentiated *in vitro* into specialized cell lineages of all three embryonic germ layers — ectodermal, mesodermal and endodermal —in the presence of physical-inducing and biological-inducing factors. So far, many promising studies have shown the therapeutic potential of differentiated derivatives of ESCs in ameliorating a range of disease in animal models. As a result, pluripotent stem cells have enormous potential for use in tissue engineering and transplantation therapy. If these cells can be induced to differentiate into a particular cell type, they may provide an almost unlimited source of cells for transplantation for the treatment of many devastating degenerative diseases such as diabetes, Parkinson's disease and Alzheimer's disease (Biswas, A. et al., 2007, Stem Cells Dev 16(2): 213-22; Kim, D. S. et al., 2007, Cell Transplant 16(2): 117-23; Zimmermann, W. H. et al., 2007, Trends Cardiovasc Med 17(4): 134-40).

Until recently pluripotent stem cell lines could be established only from preimplantation embryos. Besides ethical considerations on the use of human embryos, differentiated progeny of individual human ES cell lines would be recognized as foreign by the immune system of most recipients. Over the last year, however, various reports provided evidence that it is possible to reprogram differentiated mouse and human skin fibroblasts into induced pluripotent stem (iPS) cells (Hanna, J., et al. (2007). Science 318(5858): 1920-3; Meissner, A., et al. (2007). Nat Biotechnol 25(10): 1177-81; Nakagawa, M., et al. (2007). Nat Biotechnol.; Okita, K., et al. (2007). Nature 448(7151): 313-7; Takahashi, K., et al. (2007Cell 131(5): 861-72; Wernig, M., et al. (2007). Nature 448(7151): 318-24; Yu, J., et al. (2007). Science 318(5858): 1917-20; Park, I. H., et al. (2008). Nature 451 (7175): 141-6). Murine iPS cell lines were derived from fibroblasts upon infection with retroviral expression vectors for the four proteins OCT4, SOX2, c-MYC and KLF4. A small number of transduced cells (0.001-0.1 %) forms colonies that become morphologically similar to pluripotent stem cells. These murine iPS cell lines were found to be similar to naturally-isolated ES cell lines in terms of morphology, stem cell marker expression, overall gene expression and their *in vitro* and *in vivo* differentiation capacity. In later reports also human iPS cells could be derived from adult dermal fibroblasts and other primary cells upon infection with viral OCT4, SOX2, c-MYC and KLF4 expression vectors without selection for pluripotent cells. Using the same factors, human iPS cells were derived from fetal, neonatal and adult primary cells, including dermal fibroblasts isolated from a skin biopsy of a healthy subject. These cells expressed human ES cell markers, could differentiate into neuronal and cardiac cells and formed teratomas in SCID mice. A further publication reported the generation of mouse and human iPS cells from fibroblasts without the use of c-MYC at reduced efficiency. Alternatively, human fibroblasts were reprogrammed into iPS cells by retroviral expression of OCT4, SOX2, NANOG and LIN28. In a model gene therapy experiment iPS cells derived from autologuous skin of a sickle cell anemia mouse mutant were genetically corrected, differentiated into hemopoetic progenitors and used for cell therapy. Taken together, these results showed that ES cell-like, pluripotent iPS can be reproducibly generated from adult somatic cells using retroviral vectors that stably integrate multiple copies of expression units for reprogramming factors into various locations of the genome. Despite the initially high infection rate of ∼50% of the primary cells with retroviral reprogramming vectors only one of several thousand cells successfully completes the reprogramming process over the period of 3 -4 weeks. The reasons for the extremely low frequency and long duration of the reprogramming process are presently unknown. Since established iPS cell lines contain more retroviral integrations than the average of infected primary cells it has been proposed that reprogramming may require the inactivation of specific endogenous genes by vector integration.

Although patient-specific iPS cell lines would have great potential for medical use the existing technology to derive iPS cells has severe limitations:
I. Retroviral vectors used to introduce and express reprogramming factors randomly integrate into the genome in multiple copies, preferably into the vicinity or into active endogenous genes and may cause activating or inactivating mutations of cancer or tumor suppressor genes, respectively. Such genetic modifications and the continuous presence of retroviral vectors in iPS cells may lead to the development of cancerous cells from their differentiated progeny upon transplantation.
II. The continuous presence of sequences of expression vectors for reprogramming factors, e.g., the coding sequences for the reprogramming factors and their regulatory sequences, could interfere with the proper differentiation and/or function of iPS derived cells and may limit their utility for regenerative medicine.

Therefore, the technical problem underlying the present invention was to identify alternative and/or improved means and methods that allow for generation of induced pluripotent stem (iPS) cells.

The solution to this technical problem is achieved by providing the embodiments
characterized in the claims.
Accordingly, the present invention relates in a first embodiment to a DNA molecule comprising:
(a) a first DNA sequence comprising:
   (aa) a coding sequence giving rise upon transcription to a factor that contributes to the reprogramming of a somatic cell into an induced pluripotent stem (iPS) cell;
   (ab) a promoter mediating the transcription of said coding sequence; and
   (ac) two sequence motifs that mediate excision of (aa) and/or (ab) from the DNA molecule, wherein one sequence motif is positioned 5' and the other sequence motif is positioned 3' of the sequence to be excised;
(b) a second DNA sequence comprising a sequence motif that mediates site-specific integration of (a) into another DNA molecule.

The term "coding sequence" relates to a nucleotide sequence that upon transcription gives rise to the encoded product. The transcription of the coding sequence in accordance with the present invention can readily be effected in connection with a suitable promoter. Preferably, the coding sequence corresponds to the cDNA sequence of a gene that gives rise upon transcription to a target factor.

A "factor that contributes to the reprogramming of a somatic cell into an induced pluripotent stem cell" in accordance with the present invention relates to any factor that is capable of contributing to the induction of the reprogramming of somatic cells into induced pluripotent stem cells. Said contribution to the reprogramming may be in the form of, for example, changing the methylation pattern of a cell similar to an embryonic stem cell, shifting the expression profile of a cell towards the expression profile of an embryonic stem cell or affecting conformation of the aggregated nuclear DNA by modulating the histone binding similar to an embryonic stem cell wherein each of said changes may be effected either alone or in combination by a suitable reprogramming factor. Methods to identify suitable reprogramming factors comprise bisulphite genomic sequencing, RT-PCR, real-time PCR, microarray analysis, karyotype analysis, teratoma formation, alkaline phosphatase staining, all of which are well-known to the person skilled in the art and are, for example described in Okita, K., et al. (2007), Nature 448(7151): 313-7; Park, I. H., et al. (2008), Nature 451(7175): 141-6; Takahashi, K., et al. (2007), Cell 131(5): 861-72; Wernig, M., et al. (2007), Nature 448(7151): 318-24; Takahashi, K. et al. (2007), Nat Protoc 2(12): 3081-9; or Hogan, B., et al. (1994), "Manipulating the Mouse Embryo: A Laboratory Manual", Cold Spring Harbour Press. Reprogramming factors include, but are not limited to, for example, certain types of vertebrate transcription factors including OCT4, SOX2, SOX1, SOX3, NANOG, c-MYC, n-MYC,L-MYC, KLF1, KLF2, KLF4, KLF5, LIN28, and the like, or mutants thereof with retained reprogramming capabilities. Other vertebrate transcription factors known in the art are also suitable, for example, ECAT1 (ES cell associated transcript 1) or FBOX15 (F-box protein 15).

The term "reprogramming" as used in accordance with the present invention relates to the process of changing the geno- and phenotypical profile of a cell that results in a cell that is geno- and phenotypically similar to an embryonic stem cell. Said changes comprise, for example, changes in the methylation pattern, shifts in the expression profile or conformational changes of the aggregated nuclear DNA.

A "somatic cell" as used herein is any cell other than a germ line cell. Preferably said cell is a cell that is proliferative and exhibits a normal karyotype, for example, a fibroblast, a hepatocyte, a macrophage or an epithelial cell, for example, a gastric epithelial cell. Somatic cells in accordance with the present invention are preferably mammalian somatic cells.

An "induced pluripotent stem (iPS) cell" is a cell that exhibits characteristics similar to embryonic stem cells (ESCs). Said characteristics include, for example, unlimited self renewal in vitro, a normal karyotype, a characteristic gene expression pattern including stem cell marker genes like Oct3/4, Sox2, Nanog, alkaline phosphatase (ALP) and stem cell-specific antigen 3 and 4 (SSEA3/4), and the capacity to differentiate into specialized cell types (Hanna, J., et al. (2007). Science 318(5858): 1920-3; Meissner, A., et al. (2007). Nat Biotechnol 25(10): 1177-81; Nakagawa, M., et al. (2007). Nat Biotechnol.; Okita, K., et al. (2007). Nature 448(7151): 313-7; Takahashi, K., et al. (2007Cell 131(5): 861-72; Wernig, M., et al. (2007). Nature 448(7151): 318-24; Yu, J., et al. (2007). Science 318(5858): 1917-20; Park, I. H., et al. (2008). Nature 451(7175): 141-6). The pluripotency of murine iPS cells can tested by *in vitro* differentiation into neural, glia and cardiac cells and the production of germline chimaeric mice through blastocyst injection. Human iPS cells lines can be analyzed through *in vitro* differentiation into neural, glia and cardiac cells and their *in vivo* differentiation capacity can be tested by injection into immunodeficient SCID mice and the characterisation of resulting tumors as teratomas.

The term "promoter" relates to promoters which are functional in a eukaryotic cell and mediate the expression of said factor contributing to the reprogramming within said cell. The structure and function of eukaryotic promoters are well-known to the person skilled in the art and described, for example, in "Molecular Cell Biology", Lodish et al. (eds), W.H.Freeman&Co, New York. The promoters may be RNA polymerase II or III dependent and constitutively active or inducible, ubiquitous or gene-/tissue-specific. Further, the promoters may contain artificially introduced sequences to modify their regulatory capacity, such as, for example, enhancers, silencers, insulators, specific transcription factor binding sites or specific operator sequences like to, tet, Gal4, lac conferring inducibility to said promoter. Preferred promoters are inducible promoters functional in eukaryotic cells.

The "two sequence motifs that mediate excision" relate to specific nucleotide sequences that allow for enzyme-mediated cleavage of double stranded DNA, hence excising the DNA segment that is enclosed by said specific nucleotide sequences from a DNA molecule, and subsequent ligation of the remaining ends that do not belong to the excised DNA segment and the ends that belong to said excised DNA segment. Preferably, the sequence motifs excise (aa) and (ab), more preferred is excision of (a) from the DNA molecule. Generally, it is preferred that the sequence motifs are positioned in a way that allows for the excision of as much as possible of the DNA molecule of the invention from a target DNA molecule. In accordance with the invention, sequence motifs may be - independently from each other (under the prerequisite that one motif is located 5' and one motif is located 3' of the enclosed sequence) - positioned directly adjacent to the enclosed sequence or positioned 5' or 3' within a distance of said sequence such as, e.g., a minimal distance of 1 bp up to a distance of 5000 bp. Suitable distances thus include any distance within this range such as 50 bp, 100 bp, 250 bp, 500 bp, 1000 bp or 2500 bp. It is understood in accordance with the present invention that if only the promoter is removed the expression of the coding sequence (aa) is not possible, e.g., by endogenous cellular factors when the DNA molecule is part of the genomic DNA of a somatic cell. Preferably, such sequence motifs are recombinase recognition sequences. Said recognition sequences are recognized by enzymes capable of mediating site-specific recombination. Recombination involves enzyme-mediated cleavage of a DNA double strand and subsequent ligation of the cleaved DNA double strand to either the same or another equally cleaved DNA strand. The recombinase recognition sequence comprises a sequence motif which is specifically recognized by a recombinase and further contains a sequence motif which matches with the terminal end of a DNA molecule for subsequent ligation, wherein recognition and matching motif can be overlapping or be the same. Thus, recombination can be used to excise or introduce a DNA segment by cleavage and ligation of dsDNA. In accordance with the present invention, preferred are recombination recognition sequences which are aligned such as to only allow for the excision of the DNA segment that is flanked by said sequence motifs when integrated into a target DNA molecule, i.e., for example, by using loxP sequences as direct repeats instead of inverted repeats. Suitable sequence motifs are well-known to the skilled person and include without limitation phage recombinase recognition motifs, for example, wild-type loxP sequences or mutated loxP sequences that have retained recombination capabilities. Further examples include FRT sequences, rox sequences (Sauer, B. and J. McDermott (2004), Nucleic Acids Res 32(20): 6086-95), an attB or attP sequence recognizable by TP901 or phiBT1 integrase or mutated sequences thereof having retained their recombination capabilities.

The "sequence motif that mediates site-specific integration" as used in accordance with the present invention relates to a specific nucleotide sequence that allows for integration of a DNA molecule into another DNA molecule, for example, genomic DNA, at a specific locus. Said integration may be mediated by, e.g., homologous recombination or enzyme-mediated recombination, for example, by any recombinase or integrase. Preferably, site-specific integration is effected by the action of integrases which act unidirectional. Said integrases therefore increase the likelihood of persistent integration as they cannot also mediate excision of the integrated sequence in contrast to bidirectional recombinases. Accordingly, preferred is a sequence motif recognizable by an integrase. Preferred are phage integrases that allow for unidirectional integration of the DNA molecule into a target DNA molecule at, for example, pseudo recognition sites as outlined infra.

Moreover, the DNA molecule may comprise in any of the DNA sequences additional sequences that encode, for example, selection markers conferring antibiotic resistance or reporter genes such as, for example fluorescent proteins or other reporter genes well-known in the art.

The above explanations apply mutatis mutandis to other embodiments herein.

Despite the potential therapeutic and research application offered by induced pluripotent stem cells, no successful efforts have been made up to date that optimize the reprogramming procedure for somatic cells to exploit their therapeutic value.

The present inventors have developed a novel approach that enables to reprogram adult somatic cells into pluripotent stem cells upon the targeted genomic or extrachromosomal introduction of reprogramming factor expression vectors and that enables to eliminate the foreign gene expression elements from established iPS cells by the use of site-specific DNA recombinases. Hence, described herein is a reprogramming system for somatic cells alternative to the use of randomly integrating viral vectors, which does not lead to the disturbance of the integrity of endogenous genes and that enables to establish pluripotent stem cell lines of somatic origin that are free of exogenous gene expression elements. Such an alternative reprogramming system particularly impacts all those applications which require the differentiation of iPS cells into specialized cell types for medical purposes.
The present inventors successfully tackle the drawbacks of the currently employed methods known in the art for generating iPS cells by the introduction of an inducible expression vector preferably for the OCT4, SOX2, c-MYC and KLF4 proteins into somatic cells that is either replicating extrachromosomally as described below or that is integrated into selected chromosomal positions, not interfering with the integrity of endogenous genes, by the action of site-specific DNA recombinases or by nuclease-stimulated targeted transgene insertion. Upon the reprogramming of somatic cells into iPS cells the vector is not any more required for their maintenance and removed by site-specific DNA recombination. The present invention provides the following advantages over previous approaches for generating iPS cells: The DNA molecule as part of a vector can be integrated into the genome at a specific, selected genomic location at which the integrity of an endogenous gene is not disturbed or the vector is not integrated at all into the genome; it can function as a single vector copy that contains all required expression elements and be regulated such that the expression level of reprogramming factors can be adapted; and further be removable from established iPS cell lines.

In conclusion, the important aspect of medicine and medical research to be able to derive patient-specific pluripotent stem cell lines of somatic origin is successfully addressed. The methods presented herein enable to derive pluripotent stem cell lines that do not contain modifications in endogenous protein coding genes and that are, once established, free of foreign genetic elements used for gene expression.

The development of reprogramming procedures as shown herein avoid the use of randomly integrating, e.g. retroviral vectors, and that lead to the establishment of ultimately vector-free iPS cell lines and can therefore fully exploit the medical and research potential of human iPS cell lines.

In a further embodiment, the invention relates to a DNA molecule comprising:
(a) a first DNA sequence comprising:
   (aa) a coding sequence giving rise upon transcription to a factor that contributes to the reprogramming of a somatic cell into an induced pluripotent stem (iPS) cell; and
   (ab) a promoter mediating the transcription of said coding sequence;
(b) a second DNA sequence comprising:
   (ba) a sequence motif that mediates extrachromosomal self-replication of the DNA-molecule; and
   (bb) two sequence motifs that mediate excision of at least said sequence motif of (ba) from the second DNA sequence (b), wherein one sequence motif is located 5' of (ba) and the other sequence motif 3' of (ba).

The term "extrachromosomal self-replication" as used herein relates to the capability of a DNA molecule that resides extrachromosomally in a somatic cell to replicate itself. For example, a vector transfected into a eukaryotic cell will not be duplicated prior to cell division in contrast to the chromosomal DNA. Hence, after cell division has taken place only one of the resulting cells still carries the vector, i.e. the vector is gradually lost during multiple cell divisions. However, having a sequence .motif incorporated that allows for self-replication which is triggered, for example, by the same factors that induce duplication of the chromosomal DNA also the vector would be duplicated and the cells resulting after cell division are likely to both carry a copy of the vector.

In accordance with the present invention the sequence motif of (ba) may be any sequence motif that enables extrachromosomal self-replication of a vector in eukaryotic cells. Preferred are viral sequence motifs that allow episomal amplification during the latent phase of the virus thus enabling long-term episomal persistence. Such sequence motifs include, but are not limited to, the EBNA1 and oriP elements derived from the Epstein Barr Virus (EBV) (Bornkamm, G. W., (2005). Nucleic Acids Res 33(16): e137; Satoh, E., et al. (1997), Biochem Biophys Res Commun 238(3): 795-9; Sclimenti, C. R., et al. (1998), Curr Opin Biotechnol 9(5): 476-9), the E1 and E2 proteins and the MO and MME elements of the bovine papilloma virus (BPV) genome (Piirsoo, M, et al. (1996), EMBO J 15(1): 1-11; Ohe, Y. et al. (1995), Hum Gene Ther 6(3): 325-33), the large T antigen and origin of replication of the Polyoma virus (Camenisch, G. et al. (1996), Nucleic Acids Res 24(19): 3707-13), or mutants thereof that have retained their capability of episomal amplification.

The present inventors have been able to design a further DNA molecule combining - when part of a vector - the advantages of the DNA molecule first described herein when generating iPS cells and the further advantage of not having to manipulate the chromosomal DNA in order to site-specifically integrate DNA sequences. In contrast, the DNA molecule - when part of a vector - can be stably maintained extrachromosomally, whereas non-replicating vectors as outlined-above require chromosomal integration. The sequence motifs (bb) enclosing the sequence motif (ba) that mediates extrachromosomal self-replication allow for excision of the latter after reprogramming has taken place resulting in a vector that cannot replicate itself and is therefore gradually lost. Hence, an iPS cell line without foreign genetic material can be established. The inventors exemplarily show this capability in the non-limiting Example 2.

In a preferred embodiment of the DNA molecule of the invention, said coding sequence of (aa) is selected from the group consisting of Oct, Sox, Klf, Myc, Nanog and Lin coding sequences or combinations thereof.

The coding sequence of, for example, murine Oct3/4, Sox2, Nanog, c-Myc, Klf4 and Lin28 can be found in SEQ ID NOs: 1, 5, 9, 13, 17 and 21, respectively. The protein sequence of murine OCT3/4, SOX2, NANOG, c-MYC, KLF4 and LIN28 can be found in SEQ ID NOs: 2, 6, 10, 14, 18 and 22, respectively. The coding sequence of human Oct3/4, Sox2, Nanog, c-Myc, Klf4 and Lin28 can be found in SEQ ID NOs: 3, 7, 11, 15, 19 and 23, respectively. The protein sequence of human OCT3/4, SOX2, NANOG, c-MYC, KLF4 and LIN28 can be found in SEQ ID NOs: 4, 8, 12, 16, 20 and 24, respectively. The skilled person is in the position to determine the coding sequences of reprogramming factors for any target species using methods well-known in the art. For example, he can retrieve data relating to sequence and function from databases such as, for example, the databases maintained by the National Center for Biotechnology Information (NCBI) and accessible via the World Wide Web under http://www.ncbi.nlm.nih.gov/. Further, databases for comparative genomics include without limitation, a database maintained also by the NCBI at http://www.dcode.org/, a database for protein annotations for all completely sequenced organisms accessible at hftp://supfam.org/SUPERFAMILY/, a database comprising genome information for various species accessible at hftp://www.cbs.dtu.dk/services/GenomeAtlas/, or a database comprising gene clusters accessible at http://phigs.jgi-psf.org/. Said databases allow the skilled person to identify coding sequences for reprogramming factors in other species starting from the sequences known for mice and humans by, for example, performing cross-species sequence alignments to identify homologuous genes.

Several, only recently published scientific articles (Hanna, J., et al. (2007). Science 318(5858): 1920-3; Meissner, A., et al. (2007). Nat Biotechnol 25(10): 1177-81; Nakagawa, M., et al. (2007). Nat Biotechnol.; Okita, K., et al. (2007), Nature 448(7151): 313-7; Takahashi, K., et al. (2007), Cell 131(5): 861-72; Wernig, M., et al. (2007). Nature 448(7151): 318-24; Yu, J., et al. (2007). Science 318(5858): 1917-20; Park, I. H., et al. (2008). Nature 451(7175): 141-6) have shown that transcription factors belonging to the Oct, Sox, Klf, Myc, Nanog and Lin families are particularly capable of inducing reprogramming in murine as well as human somatic cells. Independently, the research groups have worked on identifying effective combinations of said factors and elucidating the contribution of the single factors to the reprogramming event that is triggered upon concerted expression of said factors within a somatic cell.

Accordingly, in another preferred embodiment of the DNA molecule of the invention, said coding sequence of (aa) is selected from the group consisting of Oct3/4, Sox1, Sox2, Sox3, Sox15, Sox18, Klf1, Klf2, Klf4, Klf5, n-Myc, I-Myc, c-Myc, Nanog and Lin28 coding sequences or combinations thereof.

Said coding sequence of (aa) comprises in further preferred embodiment of the DNA molecule of the invention 3 coding sequences selected from the combinations of Oct3/4, a Sox and a Klf coding sequence.

In a more preferred embodiment of the DNA molecule, said coding sequence of (aa) comprises the coding sequences of Oct3/4, Sox2 and Klf4.

In a preferred embodiment of the DNA molecule, said coding sequence of (aa) comprises 4 coding sequences selected from the combinations of Oct3/4, a Sox, a Klf, and a Myc coding sequence and Oct3/4, a Sox coding sequence, Nanog and Lin28.

In a more preferred embodiment of the DNA molecule of the invention, said coding sequence of (aa) comprises 4 coding sequences selected from the combinations of Oct3/4, Sox2, Klf4, c-Myc; and Oct3/4, Sox2, Nanog, Lin28.

The promoter of the DNA molecule of the invention is in a preferred embodiment an inducible promoter.

An "inducible promoter" as used in the present invention relates to a promoter that is exerts its activity upon induction by the presence or absence of biotic or abiotic factors. Promoters that are regulated by biotic factors may be referred to as chemically-regulated factors and include, e.g., alcohol-regulated promoters, steroid-regulated promoters, metal-regulated promoters, tetracycline-regulated promoters. Promoters that are regulated by abiotic factors may be referred to as physically-regulated promoters and include, e.g., temperature-regulated promoters, light-regulated promoters or promoters that are regulated by certain gases or a change in concentration of certain gases, e.g., decrease in oxygen. Said promoters may be positive or negative regulated promoters, i.e. activated by the presence of a biotic or abiotic factor (positive mode of action) or deactivated by said factors (negative mode of action), respectively. Advantageously, the factor should i) not be naturally present in the organism or the environment of the organism, ii) only affect the expression of the reprogramming factors and iii) be easily applied and/or removed.
The DNA molecule of the present invention preferably comprises an inducible promoter. Said inducible promoter is advantageous in the sense that it allows for a timely and quantitatively regulated mode of expression of the reprogramming factors. Suitable promoter systems include without limitation, for example, an acetaldehyde inducible gene expression system (Werner, N. S., et al. (2007), Biotechnol Bioeng 96(6): 1155-66), a nicotine inducible gene expression system (Malphettes, L., et al. (2006), Metab Eng 8(6): 543-53), doxycycline inducible gene expression system (Baron, U. and H. Bujard (2000), Methods Enzymol 327: 401-21; Freundlieb, S., et al. (1999). J Gene Med 1(1): 4-12; Gossen, M. et al. (1995), Science 268(5218): 1766-9; Urlinger, S., et al. (2000), Proc Natl Acad Sci USA 97(14): 7963-8) or a macrolide inducible gene expression system (Weber, W. ef al. (2004), Methods Mol Biol 267: 451-66).

In a more preferred embodiment of the DNA molecule of the invention, the promoter (ab) is inducible by doxycycline.

Several doxycycline inducible promoter systems have been described in the art and are well-known to the person skilled in the art (Gossen, M. and H. Bujard (1992), Proc Natl Acad Sci USA 89(12): 5547-51; Deuschle, U. et al. (1995), Mol Cell Biol 15(4): 1907-14). Generally, doxycycline-regulated promoters can be activated or deactivated upon exposure to doxycycline. An inducible promoter system in accordance with the invention can be based on the binding of tet-repressor proteins to promoter regions containing tet operator sequences leading to expression of the coding sequences controlled by the promoter (Baron, U. and H. Bujard (2000), Methods Enzymol 327: 401-21; Freundlieb, S., et al. (1999), J Gene Med 1(1): 4-12; Gossen, M. and H. Bujard (1992), Proc Natl Acad Sci U S A 89(12): 5547-51; Gossen, M., et al. (1995), Science 268(5218): 1766-9; Kistner, A., et al. (1996), Proc Natl Acad Sci U S A 93(20): 10933-8; Urlinger, S., et al. (2000), Proc Natl Acad Sci U S A 97(14): 7963-8). For example, a fusion protein of the VP16 activator protein with a mutant "reverse" tet repressor (rtTA2(S)-M2) (Urlinger, S., et al., Proc Natl Acad Sci USA, 2000. 97(14): p. 7963-8) can be used to activate transcription from a bidirectional inducible promoter in the presence of doxycycline. The coexpression of a transcriptional silencer fusion protein with the wildtype tet repressor (tTS) (Freundlieb et al., J Gene Med, 1999. 1(1): p. 4-12) can be used to actively repress background promoter activity in the absence of doxycycline while in its presence the tTS protein dissociates from the promoter. By the combined use of a transcriptional activator binding to the regulated promoter in the presence of inducer and a repressor fusion protein that binds only in its absence, promoter activity can be regulated over a wide range of activity.

In a further preferred embodiment of the invention, the promoter (ab) is a bidirectional minimal promoter.

A bidirectional promoter is an artificial genetic element that initiates transcription into both directions of the DNA strand. For example, a commonly used bidirectional promoter (Baron, U., et al. (1995), Nucleic Acids Res 23(17): 3605-6) contains a central part composed of seven tet operator sequences (tetO7) that serves as binding site for transcriptional activator or repressor proteins fused to a wildtype or mutant tet repressor. The tetO7 segment is flanked at both ends with a eukaryotic minimal promoter segment, derived from the cytomegalovirus IE gene, which as such exhibits only a very low level of transcriptional activity but functions as a strong promoter upon binding of an activator protein to the tetO7 element.

In a preferred embodiment of the DNA molecule, the sequence motif of the second DNA sequence (b) is selected from the group consisting of attB, attP and an ITR (inverted terminal repeat), wherein the ITR is recognized by an adeno-associated virus (AAV) integrase.

In accordance with the present invention, "attB, attP" are sequence motifs that are recognized by bacteriophage recombinases that mediate unidirectional recombination. Phage recombinases naturally mediate recombination events between phage DNA carrying an attP sequence motif (phage attachment sequence) and bacterial DNA carrying an attB sequence motif (bacterial attachment sequence) in order to incorporate said phage DNA into said bacterial DNA. Said mechanism of integration has developed into a widely used genetic tool and is described for example in Groth, A.C. and M.P. Calos, J Mol Biol, 2004, 335(3): p. 667-78. In brief, the recombinase recombines two DNA molecules by i) cleaving them at recombinase recognition sites, in this case at the attB and attP sequence motifs, and ii) subsequently ligating the resulting ends to form a single DNA molecule, wherein each recombinase only recognizes a sequence motif, viz. attB and attP, unique to it. Upon ligation the attB and attP sequence motifs are changed to hybrid motifs, partially comprising attB and attP sequences and may be referred to as attL and attR (Landy, (1989), Annu Rev Biochem 58;913-949). In accordance with the present invention, recombinases that recognize the attB or attP sequence motif are preferably capable of mediating unidirectional recombination between the dissimilar attachment sites. The attB or attP sequences comprise wild type or mutant attB or attP sequences that have retained their capability of mediating a recombination event between each other as described supra.

In accordance with the invention an ITR (inverted terminal repeat) that is recognized by an adeno-associated virus (AAV) integrase is a sequence motif that mediates the site-specific integration of the DNA molecule of the present invention into another DNA molecule by the action of an adeno-associated viral integrase such as, for example, REP78/68 or mutants thereof that have retained the capabilty to recombine recognition sequences. Said viral integrase is capable of integrating the DNA molecule of the present invention into another DNA molecule at a locus that also carries a binding site for said integrase. For example, a naturally occurring binding site in the human genome is the so-called AAVS1 locus on chromosome 19. The method of action of said integrase, viz. cleaving and ligating, is similar to that described for phage recombinases herein-above and results in site-specific integration, for example, within the AAVS1 locus in the human genome.

The present DNA molecule can be integrated site-specifically into another DNA molecule either by introducing an attachment motif (att) or an AAVS1 sequence which can be recombined with the attachment motif or ITR of the DNA molecule of the present invention into the target DNA molecule or by taking advantage of naturally occurring attachment motifs in the target DNA molecule, e.g., commonly referred to as "pseudo attachment/recognition sites" in the case of attachement motifs. Introduction of an attachment motif or an ITR into a target DNA molecule can be achieved by methods well-known in the art (Sambrook et al., Molecular Cloning, A Laboratory Manual, Cold Spring Harbor Laboratory (1989) N.Y.), for example, homologous recombination or enzyme-mediated recombination, such as recombinase or restriction enzyme mediated cleavage of double-strand DNA with subsequent ligation.

Preferably, the DNA molecule of the present invention is site-specifically introduced into the target DNA at pseudo recognition/attachment sites. A pseudo attachment site in accordance with the present invention is a sequence motif that is partially identical to an att motif and mediates site-specific integration at a frequency that is higher than random integration into the same sequence. Preferably, said frequency of integration is at least 2-fold, at least 10-fold, or at least 100-fold, more preferred at least 1000-fold and most preferred at least 10000-fold higher than random integration into the same sequence. Several pseudo attachment sites have been identified and characterized in, e.g., the human genome for a variety of phage recombinases such as, for example, phiC31 Integrase (Calos, M. P. (2006), Curr Gene Ther 6(6): 633-45; Chalberg, T. W., et al. (2006), J Mol Biol 357(1): 28-48; Thyagarajan, B., et al. (2001), Mol Cell Biol 21(12): 3926-34), A118 Integrase (Keravala, A., et al. (2006), Mol Genet Genomics 276(2): 135-46); phiBT1 Integrase (Chen, L. et al. (2008). Hum Gene Ther 19(2): 143-52), Cre recombinase or FLP recombinase (Branda, C. S., et al. (2004). Dev Cell 6(1): 7-28). The skilled person is well-aware of the mode of action of said recombinases and suitable attB or attP motifs that can be used to mediate the site-specific integration of the DNA molecule of the present invention into a target DNA molecule.

Preferred, when employing the adeno-associated viral integrase method to site-specifically integrate the DNA molecule of the present invention into a target DNA molecule, is integration at naturally occurring integration sites. For example, in the human genome the adeno-associated virus has during evolution developed the unique ability to integrate into a 4kb region on chromosome 19, which has been termed the AAVS1 locus (Cheung et al. (1980), J Virol, 33:739-748). Integration at that site is not a site-specific integration event in the classic sense since the integration does not occur at a specified seuquence but rather in a sequence stretch of about 4kb on said chromosome. The mechanism is not yet known, but it requires the adeno-associated virus integrase and a 33 bp region of the AAVS1 locus that includes a integrase binding site and a nicking site for the integrase that resembles the terminal resolution site (trs) within the 145 base inverted terminal repeats (ITRs) of the adeno-associated virus genome (McAlister, VJ., and Owens, RA. (2007), J Virol, 81(18):9718-9726). Advantageously, integration at said locus does not affect the transcriptional activity of the target cell. Further, it has been shown that integration at this locus in human embryonic stem cells does not lead to transgene silencing as the region appears to have native insulators that protect this site from silencing and additionally, the ITR also seems to exhibit isolating activity itself (Smith et al. (2008), Stem Cells, 26:496-504). Hence, employing this strategy in the process of reprogramming of somatic cells leads to efficient expression of the site-specifically integrated reprogramming factors without being affected by transgene silencing. Alternatively, for example in other species, adeno-associated virus integrase binding sites may artificially be introduced by methods well-known by the person skilled in the art (Bakowska et al. (2003), Gene Ther.,10(19):1691-702).

In another preferred embodiment of the DNA molecule of the invention, the sequence motif of the second DNA sequence (b) comprises two sequences that flank the first DNA sequence (a) and are as a combined sequence essentially identical to a sequence at the site of integration.

The term "essentially identical" as used in accordance with the present invention is meant to comprise two sequences that combined are at least 90%, more preferred at least 95%, such as at least 98% or 99% and most preferred at least 100% (or any arbitrary number in-between) identical to a sequence at the site of integration. The skilled person is well-aware of methods to determine sequence identities. Two nucleotide or protein sequences can, for example, be aligned electronically using suitable computer programs known in the art. Such programs comprise BLAST (Altschul et al. (1990), J. Mol. Biol. 215, 403-410), variants thereof such as WU-BLAST (Altschul & Gish (1996), Methods Enzymol. 266, 460-480), FASTA (Pearson & Lipman (1988), Proc. Natl. Acad. Sci. USA 85, 2444-2448) or implementations of the Smith-Waterman algorithm (SSEARCH, Smith & Waterman (1981), J. Mol. Biol. 147, 195-197). These programs, in addition to providing a pairwise sequence alignment, also report the sequence identity level (usually in percent identity) and the probability for the occurrence of the alignment by chance (P-value). Programs such as CLUSTALW (Higgins et al. (1994), Nucleic Acids Res. 22, 4673-4680) can be used to align more than two sequences.

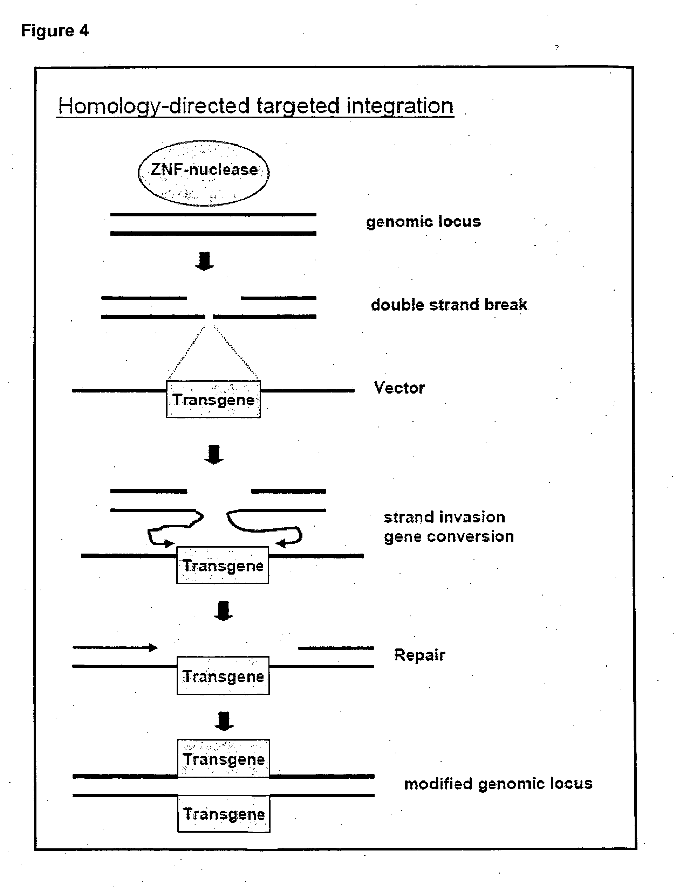

In accordance with the present invention the sequence motif of the second DNA sequence (b) that mediates site-specific integration may comprise two specific sequences, wherein said two sequences flank the DNA sequence to be integrated into another DNA molecule. Said arrangement of sequences can be employed to site-specifically introduce the flanked DNA sequence via homologous recombination when designing the flanking sequence motifs to be as a combined sequence essentially identical to the sequence at the targeted integration site. Homologous recombination is a process well-known to the skilled person in the art who is equally aware of methods taking advantage of homologous recombination to integrate a DNA molecule into another DNA molecule (Lodish et al. (2000), Molecular Cell Biology, 4th ed., W. H. Freeman and Company.). In brief, homologous recombination involves the alignment of essentially identical sequences, a crossover between said strands when aligned and subsequent cleavage and ligation of the DNA strands resulting in an exchange of sequences. For example, the DNA molecule sequence may be site-specifically introduced by a method that uses a designed zinc finger nuclease (ZNF) as described in Moehle et al. (2007), PNAS, 104, 9:3055-3060. In brief, the generally low frequency of spontaneous homologous recombination can be increased by. DNA double strand breaks that are effected by engineered zinc finger nucleases (High, K. (2005), Nature, 435:577-579). Said nucleases comprise a nuclease which is fused to a zinc finger DNA recognition motif that is designed to recognize and bind to a defined sequence on a DNA strand. Upon recognition and binding the nuclease cleaves the DNA double strand. The cleaved strand is subsequently repaired by homology-directed repair (HDR) that corresponds to the synthesis-dependent strand annealing (SDSA) model of double-strand break repair (Nassif et al. (1994), Mol Cell Biol, 14:1613-1625; Symington, LS. (2002), Microbiol Mol Biol Rev, 66:630-670) using as template a DNA molecule that displays sequence identity to the site of the double-strand break. Hence, it is shown that efficient, site-specific gene addition into a predetermined endogenous locus in cells can occur, if a ZNF-cleaved locus is provided with a template that comprises novel genetic information flanked by appropriate regions of target site homology. Said zinc finger nuclease-mediated gene insertion in contrast to other integration methods can take place at a higher frequency than random integration via homologous recombination without selection and invokes a natural process of genetic transfer most likely via the above-mentioned double-strand repair mechanism. Accordingly, the two sequence motifs flanking the first DNA sequence (a) of the DNA molecule of the present invention can be used as starting templates being essentially identical to the site of the double-strand cleavage and as a result of the repair process the first DNA sequence (a) is incorporated into the target DNA strand. This method of integration, however, does not physically integrate the first DNA molecule (a) but the latter is instead being used as a matrix. Hence, the DNA sequence (a) is added to the target DNA sequence with the matrix DNA, i.e. DNA sequence (a), physically remaining part of the template DNA (cf. Figure 4).

In a further preferred embodiment of the invention, the sequence motifs of the DNA molecule allowing excision of (aa) and/or (ab) or said sequence motif of (ba) are lox sequences.

The term "lox sequences" as used in accordance with the present invention relates to sequence motifs which are specifically recognized by the Cre recombinase, a tyrosine recombinase with type I topoisomerase activity from the P1 bacteriophage. The "lox sequences" referred to herein encompass the wild type loxP recognition sequence consisting of 34 bp wherein two 13 bp palindromes (inverted repeats) are flanking an 8 bp core region and wherein the wild type loxP recombinase recognition has the sequence of SEQ ID NO.:25. Further encompassed are mutant loxP sequences, wherein the mutant loxP sequences include sequences with not more than 8 nucleotide substitutions relative to the wild type loxP sequence of SEQ ID NO.:25. Mutant loxP sequences with 1, 2, 3, 4, 5, 6 or 7 nucleotide substitutions are deliberately envisaged. The above applies mutatis mutandis to other embodiments described herein.

As outlined herein supra, lox sequences are specifically recognized by Cre recombinases and mediate recombination events - either integration (addition) or excision (deletion) depending on the assembly of said lox sequences. Preferably, the lox sequences enclosing (aa) and/or (ab) are aligned as direct repeats instead of inverted repeats when integrated into the target DNA molecule. Equally preferred, the lox sequences enclosing (ba) are aligned as direct repeats instead of inverted repeats. Methods to delete sequences enclosed by lox sequences using the Cre/lox site-specific recombination system are well-known to the person skilled in the art and described, for example, in Branda, C. S. and S. M. Dymecki (2004), Dev Cell 6(1): 7-28; or Kwan, K. M. (2002), Genesis 32(2): 49-62.

In a preferred embodiment of the DNA molecule of the invention, the sequence motif (ba) of the invention comprises an EBNA1 and an oriP element.

The term "EBNA1" as used in accordance with the invention relates to the Epstein Barr virus (EBV) nuclear antigen 1. The viral EBNA1 protein binds to specific sites in the viral origin of DNA replication, oriP, to activate the initiation of DNA replication, enhance the expression of other viral latency proteins, and partition the viral episomes during cell division. The DNA binding domain of EBNA1 is required for all three functions, and a Gly-Arg-rich sequence between amino acids 325 and 376 is required for both the transcriptional activation and partitioning functions.

The term "oriP" as used herein relates to the latent origin of replication (oriP) sequence of the Epstein Barr virus. oriP is a 1.7-kb region of the Epstein-Barr virus (EBV) chromosome that supports replication and stable maintenance of plasmids in human cells that contain EBV-encoded protein EBNA1. Plasmids that depend on oriP are replicated once per cell cycle by cellular factors. The replicator of oriP is an approximately 120-bp region called DS which depends on either of two pairs of closely spaced EBNA1 binding sites. EBNA1 induces DNA to bend significantly when it binds, with the center of bending coinciding with the center of binding.

The EBNA1/oriP system is well-known to the skilled person and has been described, for example, in Bornkamm, G. W., (2005), Nucleic Acids Res 33(16): e137; Satoh, E., et al. (1997), Biochem Biophys Res Commun 238(3): 795-9; or Sclimenti, C. R., et al. (1998), Curr Opin Biotechnol 9(5): 476-9. Incorporation of said EBNA1/oriP elements into the DNA molecule of the invention when part of a vector has the advantage of allowing said vector to persist in the nucleus of the target cell as multi-copy episome. Further, the expression of the reprogramming factors cannot be interrupted or subjected to regulatory constraints arising from chromosomal integration.

In a more preferred embodiment of the DNA molecule of the invention, EBNA1 is flanked by a first type of lox sequences and the oriP element is flanked by a second type of lox sequences and wherein (a) said first type of lox sequences are recognized and recombined by a Cre-recombinase but not recombined with said second type of lox sequences; and (b) said second type of lox sequences are recognized and recombined by the recombinase of (a) but not recombined with said first type of lox sequences.

In accordance with the invention, the first type of lox sequences and the second type of lox sequences are not compatible for recombination with each other, but are exclusively recombinable with lox sequences with an identical sequence by the activity of the Cre recombinase. Several Cre recombinases are presently known and derived via mutagenesis from the wild type bacteriophage P1 Cre recombinase in order to change features like, for example, nuclear localization or translation efficiency in mammalian cells. The skilled person is in the position to identify suitable combinations of lox sequences to be recognized by a single Cre recombinase which can be used in accordance with the present invention.

In accordance with the present invention, EBNA1 and oriP are excised separately from the DNA molecule of the invention and subsequently the remaining DNA molecule, the EBNA1 element and the oriP element are ligated upon the action of a Cre recombinase. The resulting circularized DNA molecules are gradually lost during multiple cell divisions resulting in cells devoid of DNA molecules or vectors of the invention.

In another embodiment, the invention relates to a vector comprising the DNA molecule of the invention.

Preferably, the DNA molecules of the present invention are circularized DNA molecules such as, for example, a DNA vector. Alternatively, the DNA molecules of the invention can be incorporated into vectors by methods well-known in the art. Said circularized DNA molecules or said vectors preferably contain a region with multiple restriction enzyme recognition sites, a bacterial origin of replication and a selectable resistance, e.g. beta-lactamase, gene. Suitable vector backbones are, for example and without limitation, pBluescript (Stratagene), pNEB 193 (New England Biolabs) or similar vectors.

A vector referred to herein that is not or does not contain a DNA molecule of the invention preferably is a plasmid, cosmid or another vector conventionally used e.g. in genetic engineering. Incorporation of a nucleic acid molecule into a vector offers the possibility of introducing the nucleic acid molecule efficiently into the cells and preferably the genomic DNA of a recipient. The recipient may be a single somatic cell as described herein supra. Such a measure facilitates to express the coding sequences in the recipient. Thus, incorporation of the nucleic acid molecule into a vector opens up the way to a permanently elevated level of the encoded reprogramming factor in any cell or a subset of selected cells of the recipient.

The nucleic acid molecule may be inserted into several commercially available vectors. Non-limiting examples include vectors compatible with an expression in mammalian cells like pREP (Invitrogen), pcDNA3 (Invitrogen), pCEP4 (Invitrogen), pMC1neo (Stratagene), pXT1 (Stratagene), pSG5 (Stratagene), EBO-pSV2neo, pBPV-1, pdBPVMMTneo, pRSVgpt, pRSVneo, pSV2-dhfr, pIZD35, pLXIN, pSIR (Clontech), pIRES-EGFP (Clontech), pEAK-10 (Edge Biosystems) pTriEx-Hygro (Novagen), pCINeo (Promega), Okayama-Berg cDNA expression vector pcDV1 (Pharmacia), pRc/CMV, pcDNA1, pSPORT1 (GIBCO BRL), pGEMHE (Promega), pSVL and pMSG (Pharmacia, Uppsala, Sweden), pRSVcat (ATCC 37152), pSV2dhfr (ATCC 37146) or pBC12MI (ATCC 67109).

The nucleic acid molecule referred to above may also be inserted into vectors such that a translational fusion with another nucleic acid molecule is generated. The vectors may also contain an additional expressible polynucleotide coding for one or more chaperones to facilitate correct protein folding.

For vector modification techniques, see, for example, "Molecular Cloning: A Laboratory Manual" by Sambrook et al. (Cold Spring Harbour Laboratory Press) or "Current Protocols in Molecular Biology" by Ausubel et al. (Wiley and Sons, Inc). Generally, vectors can contain one or more origin of replication (ori) and inheritance systems for cloning or expression, one or more markers for selection in the host, e. g., antibiotic resistance, and one or more expression cassettes.

The coding sequences inserted in or part of the vector can e.g. be synthesized by standard methods, or isolated from natural sources. Ligation of the coding sequences to transcriptional regulatory elements and/or to other amino acid encoding sequences can be carried out using established methods. Transcriptional regulatory elements (parts of an expression cassette) ensuring expression in eukaryotic cells are well known to those skilled in the art. These elements comprise regulatory sequences ensuring the initiation of the transcription (e. g. translation initiation codon, promoters, enhancers, and/or insulators), internal ribosomal entry sites (IRES) (Owens Proc. Natl. Acad. Sci. USA 2001, 98: 1471) and optionally poly-A signals ensuring termination of transcription and stabilization of the transcript. Additional regulatory elements may include transcriptional as well as translational enhancers, and/or naturally-associated or heterologous promoter regions. Preferably, the nucleic acid molecule is operatively linked to such expression control sequences allowing expression in eukaryotic cells. The vector may further comprise nucleotide sequences encoding secretion signals as further regulatory elements. Such sequences are well known to the person skilled in the art. Furthermore, depending on the expression system used, leader sequences capable of directing the expressed polypeptide to a cellular compartment may be added to the coding sequence of the polynucleotide of the invention. Such leader sequences are well known in the art.

Possible examples for regulatory elements ensuring the initiation of transcription comprise the cytomegalovirus (CMV) promoter, SV40-promoter, RSV-promoter (Rous sarcome virus), the lacZ promoter, the gai10 promoter, human elongation factor 1a-promoter, CMV enhancer, CaM-kinase promoter, the Autographa californica multiple nuclear polyhedrosis virus (AcMNPV) polyhedral promoter or the SV40-enhancer. Examples for further regulatory elements in prokaryotes and eukaryotic cells comprise transcription termination signals, such as SV40-poly-A site or the tk-poly-A site or the SV40, lacZ and AcMNPV polyhedral polyadenylation signals, downstream of the polynucleotide. Moreover, elements such as origin of replication, drug resistance gene, regulators (as part of an inducible promoter) may also be included. Additional elements might include enhancers, Kozak sequences and intervening sequences flanked by donor and acceptor sites for RNA splicing. Highly efficient transcription can be achieved with the early and late promoters from SV40, the long terminal repeats (LTRs) from retroviruses, e.g., RSV, HTLVI, HIVI, and the early promoter of the cytomegalovirus (CMV). However, cellular elements can also be used (e.g., the human actin promoter).

The co-transfection with a selectable marker such as dhfr, gpt, neomycin, hygromycin allows the identification and isolation of the transfected cells. The transfected nucleic acid can also be amplified to express large amounts of the encoded (poly)peptide. The DHFR (dihydrofolate reductase) marker is useful to develop cell lines that carry several hundred or even several thousand copies of the gene of interest. Another useful selection marker is the enzyme glutamine synthase (GS) (Murphy et al., Biochem J. 1991, 227:277; Bebbington et al., Bio/Technology 1992, 10:169). Using these markers, the mammalian cells are grown in selective medium and the cells with the highest resistance are selected. As indicated above, the expression vectors will preferably include at least one selectable marker. Such markers include dihydrofolate reductase, G418 or neomycin resistance for eukaryotic cell culture. The same applies mutatis mutandis to other embodiments described herein.

Examples of the genetic design of suitable vectors in accordance with specific embodiments of the invention are provided without limitation in the example section.

In a further embodiment, the invention relates to a vector comprising the other DNA molecule of the invention.

In another embodiment the invention relates to a method for assembly of a vector of the invention comprising the step of
(I) integrating:
   (Ia) a sequence comprising the sequences (aa), (ab), (ac) and (b) of the invention; or
   (Ib) a sequence comprising the sequences (aa), (ab), (ba) and (bb) of the invention,
      either individually or combined as a contiguous sequence into a vector sequence; or
(II) circularizing a contiguous sequence comprising the sequences of (Ia) or (Ib).

Assembly of a vector relates in accordance with the present invention to the manufacture a vector of the invention by bringing together the specific sequences that make up said vector and define its functional properties. The composition of vectors of the invention is described herein supra as well as their functional properties. Means and methods for integrating or circularizing and generally designing and assemblying vectors are standard methods well-known in the art and described in, for example, Sambrook et al., Molecular Cloning, A Laboratory Manual, Cold Spring Harbor Laboratory (1989) N.Y., or in "Current Protocols in Molecular Biology" by Ausubel et al. (Wiley and Sons, Inc). The single sequences may be obtained by methods well-known to the skilled person, for example synthetic production or partial or complete recovery from biological sources.
The method of assembly may be used, for example, in a modular assembly approach to design and manufacture custom reprogramming vectors. Starting from an established vector assembly one can deliberately determine either a specific sequence alone, a combination of sequences or the entire sequence that is available for customization. Said modular approach for manufacturing the vector of the invention is particularly advantageous in view of minimized time and cost of assembling readily available single components. Further, in view of the rapidly growing diversity of experimental setups and the demand for individualized though standardized genetic tools said method of assembly may address the specific needs of both - industry and academia.

The invention also relates to a somatic cell comprising the DNA molecule of the invention or the vectors of the invention.

Suitable somatic cells have been described herein supra. Further, methods to introduce foreign DNA into said cells are well-known to the skilled person and are, for example, described in Sambrook et al., Molecular Cloning, A Laboratory Manual, Cold Spring Harbor Laboratory (1989) N.Y. and herein-below.

In another embodiment, the invention relates to a method to generate an induced pluripotent stem (iPS) cell comprising the steps of:
(i) introducing the DNA molecule or the vector of the invention into a somatic cell;
(ii) allowing the DNA molecule or the vector of step (i) to integrate into the genomic DNA of said somatic cell; and
(iii) excising the sequence that is enclosed by the two sequence motifs of (ac) from the DNA molecule,
wherein step (iii) is performed after reprogramming of said somatic cell has taken place.

The DNA molecule or the vector of the invention can be introduced in a cell by standard methods well-known to the skilled person in the art and described, for example, in Gene Delivery to Mammalian Cells: Nonviral Gene Transfer Techniques (Methods in Molecular Biology), W. C. Heiser (Editor), Humana Press; 1 edition, 2003. Transfection methods, i.e. the introduction of foreign DNA into a cell, include, for example, calcium phosphate transfection, DEAE-dextran transfection, electroporation, lipofection, heat shock, magnetofection, nucleofection, use of a gene gun or microinjection. Due to the low rate of reprogramming events taking place in transfected cells it is advantageous to rely on an efficient transfection method. Hence, the DNA molecule or the vectors of the present invention are preferably introduced into a somatic cell by a method achieving high transfection efficiency. For example, transfection efficiencies of at least 1%, at least 10%, or at least 25% are preferred. Suitable methods include, for example, lipofection, electroporation, nucleofection, viral vector infection or magnetofection. The same applies to other embodiments described herein - in modified form where applicable.

Somatic cells to be used in the method of the invention can be derived from existing cells lines or obtained by various methods including, for example, obtaining tissue samples in order to establish a primary cell line. Methods to obtain samples from various tissues and methods to establish primary cell lines are well-known in the art (Jones GE, Wise CJ., "Establishment, maintenance, and cloning of human dermal fibroblasts." Methods Mol Biol. 1997;75:13-21). Suitable somatic cell lines may also be purchased from a number of suppliers such as, for example, the American tissue culture collection (ATCC), the German Collection of Microorganisms and Cell Cultures (DSMZ) or PromoCell GmbH, Sickingenstr. 63/65, D-69126 Heidelberg.

Site-specific integration of said DNA molecule or said vectors of the invention into the genomic DNA of said cell in accordance with the invention depends on the particular architecture of the DNA molecule of the invention, i.e. comprising sequence motifs that mediate integration via homologous recombination or comprising sequence motifs that mediate integration via recombinases.

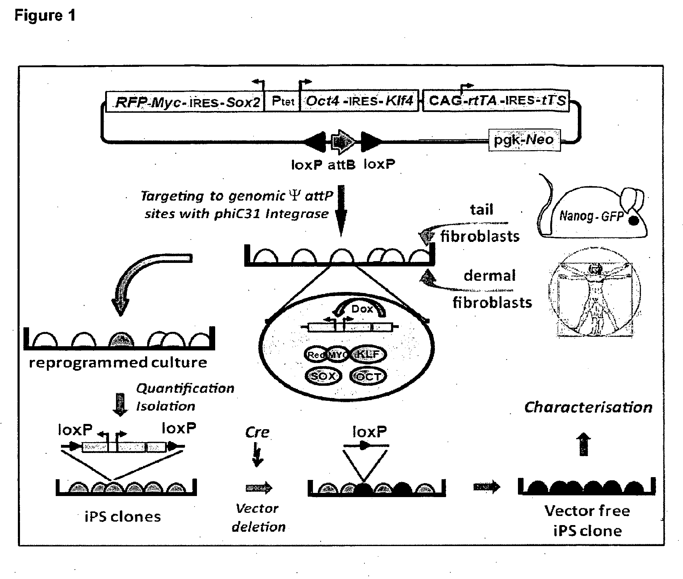

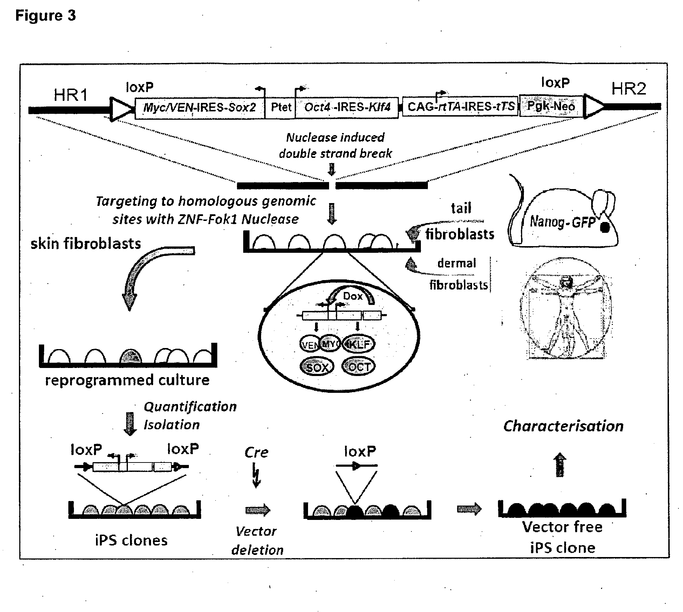

Site-specific integration via homologous recombination can be achieved via ZNF-nuclease induced DNA double strand breaks that trigger the endogenous cellular DNA repair mechanism resulting in gene addition as described herein above supra and in "Targeted gene addition into a specified location in the human genome using designed zinc finger nucleases" by Moehle, E. A., et al. (2007), Proc Natl Acad Sci U S A 104(9): 3055-60. The ZNF-nuclease is preferably added to the cell by cotransfection of an expression vector for said ZNF-nuclease and subsequent expression of said ZNF-nuclease. Alternatively, said target cell can be transfected successively in any order by said DNA molecules or vectors. Still another possibility is the transfection of mRNA coding for the ZNF-nuclease or recombinant ZNF-nuclease protein. Methods to design and produce said ZNF-nuclease are well-known to the person skilled in the art and described, for example, in Pavletich et al. (1991), Science, 252:809-817; Choo et al. (1994), Nature, 372:642-645; Pabo et al. (2001), Annu Rev Biochem, 70:313-340; Reik et al. (2002), Curr Opin Genet Dev, 12:233-242; and High KA. (2005), Nature, 435:577-579. Exemplarily, a method to generate iPS cell via homologous recombination-mediated site-specific integration is described in Example 3 (cf. also Fig. 3 for a schematic overview). Preferably, the DNA molecule is circularized or part of a vector.
Site-specific integration via recombinases - the mechanism of action has been described herein supra - can be achieved by supplying said recombinases to the target cell comprising a circularized DNA molecule or a vector of the invention. This can be achieved, for example, by cotransfection of an expression vector for said recombinase and subsequent expression of said recombinase. Alternatively, said target cell can be transfected successively in any order by said DNA molecule or vectors. Said expression vector may reside extrachromosomally or be integrated into the genomic DNA of said target cell. Also, the recombinase may be exogenously added to the cell and, upon uptake, exert activity within the target cell.
The person skilled in the art is aware of conditions generally suitable for uptake of proteins, including enzymes such as recombinases, into cells and methods to enhance said uptake as regards rate and amount wherein said enhancement may include artificially modifying proteins (cf., e.g., Patsch et Edenhofer, (2007), Handb Exp. Pharmacol., 178, 203-232). A further possibilty is to transfect mRNA coding for a recombinase. In accordance with the invention as described herein, iPS cells may be generated by introducing a DNA molecule or vector carrying all coding sequences of reprogramming factors intended to be expressed or several DNA molecules carrying any number of coding sequences of said reprogramming factors. Preferred is a DNA molecule or a vector that carries coding sequences of all reprogramming vectors, therefore only one DNA molecule or vector has to be introduced into a somatic target cell. Exemplarily, a method to generate iPS cell via recombinase mediated site-specific integration is described in Example 1 (cf. also Fig. 1 for a schematic overview).

Excision of sequences enclosed by sequence motifs mediating the excision event can be effected by the action of recombinases as described above in detail. Preferably, the gene encoding said recombinase has been introduced into the cell according to methods well-known in the art and maintained extrachromosomally, for example, on a plasmid or other vector, or stably integrated into the genome and can thus be expressed. Further, the recombinase may be exogenously added as described supra.
Excision of the target sequence in accordance with the invention is to be effected only after reprogramming has taken place. Said time point of effected reprogramming can be determined by several methods as described herein-above, generally by assessing the genotypic and phenotypic similarity to embryonic stem (ES) cells.
Further, cell culture methods, such as, for example, media constituents, marker choice and selection, cell quantification and isolation, are methods well-known in the art and described, for example, in "Practical Cell Culture Techniques", Boulton et Baker (eds), Humana Press (1992), ISBN 0896032140; "Human Cell Culture Protocols", Gareth E. Jones, Humana Press (1996), ISBN 089603335X and exemplarily in the example section. Culture conditions vary from cell-type to cell-type and moreover, can result in different phenotypes being expressed for a particular cell-type. Generally, cells are grown and maintained at an appropriate temperature and gas mixture, i.e. typically 37°Celsius, 5% CO₂, in growth media (a) as irrigating, transporting and diluting fluid while maintaining intra- and extra-cellular osmotic balance, (b) that provides cells with water and certain bulk inorganic ions essential for normal cell metabolism, (c) which - combined with a carbohydrate, such as glucose - provides the principle energy source for cell metabolism and (d) which provides a buffering system to maintain the medium within physiologic pH range, i.e. cells are kept viable. The recipe of growth media varies greatly depending on cell-type and contains, for example and without limitation, growth factors, nutrient components, glucose, buffers to maintain pH and antifungizides and -biotics. Methods for culturing and maintaining cells in culture are well-known in the art; growth media and other cell culture related material as well as instructions and methods for successful culturing of cells can, for example, be obtained at Sigma-Aldrich or Invitrogen. The culture conditions for iPS cells are the same as established for embryonic stem cells of the corresponding species and are well-known to the person skilled in the art.

The above applies mutatis mutandis to other embodiments described herein.

In an embodiment, the invention relates to a further method to generate an induced pluripotent stem cell comprising the steps of:
(i) introducing the DNA molecule or the vector of the invention into a somatic cell; and
(ii) excising the sequence motif (ba) from the DNA molecule,
wherein step (ii) is performed after reprogramming of said somatic cell has taken place.

In accordance with the present invention iPS cells can be generated without site-specific integration into the genome of the target cell. Instead, the DNA molecule of the invention circularized or part of a vector may be functionally maintained extrachromosomally. Design and function of sequences conferring said capability of extrachromosomal maintenance have been described herein supra and are moreover well-known in the art. Preferably, the DNA molecule is circularized or part of a vector.

Furthermore, the invention relates to an induced pluripotent stem cell obtainable by the method of the invention.

The invention further relates to a kit comprising the DNA molecule of the invention, a sequence comprising the sequences (aa), (ab), (ac) and (b) of the invention, a sequence comprising the sequences (aa), (ab), (ba) and (bb) of the invention, the vector of the invention, or the induced pluripotent cell of the invention.

The various components of the kit may be packaged in one or more containers such as one or more vials. The vials may, in addition to the components, comprise preservatives or buffers for storage, media for maintenance and storage, e.g. ES cell media, DMEM, MEM, HBSS, PBS, HEPES, hygromycin, puromycin, Penicillin-Streptomycin solution, gentamicin inter alia. Advantageously, the kit further comprises instructions for use of the components allowing the skilled person to conventiently work, e.g., various embodiments of the invention. Any of the components may be employed in an experimental setting. For example, the DNA molecule, the combination of sequences or the vector may be used to study the reprogramming of somatic cells into induced pluripotent stem cells. In general, the induced pluripotent stem cells of the invention may be used in any experiment instead of ES cells. For, example, the induced pluripotent stem cells may be used to study (re-)differentiation into other cells such as, e.g., nerve cells, muscle cells or blood cells. Alternatively, the cells may be used to establish cell culture conditions for maintenance or differentiation of iPS cells of the invention.

Further, the invention relates to a cell line or cell culture collection comprising the induced pluripotent stem cell of the invention.

A cell culture collection in accordance with the invention comprises at least one iPS cell line comprising at least 10², such as 10³, 10⁶, 10¹² cells. The cell culture collection comprises at least 2 different iPS cell lines, more preferred 10, 50, or 100, and most preferred more than 1000 different cell lines. Preferably, the cell culture collection maintains a variety of iPS cells from various species and reprogrammed from various somatic cell populations. Exemplary somatic cells have been described herein above. Further, the cell culture collection also maintains differentiated cells originating from said iPS cells. Also, preferred is a tissue culture collection, wherein the tissues are obtainable by differentiating said iPS cells and allowing the differentiated cells to form a tissue. Preferably, the cells are maintained under standardized conditions as is general practice in cell culture facilities. Suitable methods for establishing, maintaining, propagating and differentiating cells are well-known to the skilled person and described, e.g. in "Practical Cell Culture Techniques", Boulton et Baker (eds), Humana Press (1992), ISBN 0896032140; "Human Cell Culture Protocols", Gareth E. Jones, Humana Press (1996), ISBN 089603335X.

In another embodiment, the invention also relates to a method to generate a transgenic non-human animal comprising the steps of the above methods to generate an induced pluripotent stem cell of the invention and the further steps of:
(i) introducing the induced pluripotent stem cells into a non-human blastocyst;
(ii) transferring the blastocyst into the uterus of a female non-human animal; and
(iii) allowing the blastocyst to develop into an embryo.

The term "transgenic non-human animal" as used in accordance with the invention relates to an animal in which there has been a deliberate modification of its genome by methods described herein.

The method of the invention to generate a transgenic non-human animal is preferably carried out according to methods that have been established for generating transgenic non-human animals by the use of embryonic stem cells, however, replacing the embryonic stem cells with iPS cells. Said methods are well-known in the art (Hogan, B., R. Beddington, et al. (1994), "Manipulating the Mouse Embryo: A Laboratory Manual", Cold Spring Harbour Press; Hanna, J., et al. (2007), Science 318(5858): 1920-3; Meissner, A., et al. (2007), Nat Biotechnol 25(10): 1177-81; Nakagawa, M., et al. (2007), Nat Biotechnol.; Okita, K., et al. (2007), Nature 448(7151): 313-7; Takahashi, K., et al. (2007), Cell 131(5): 861-72; Wernig, M., et al. (2007), Nature 448(7151): 318-24; Yu, J., et al. (2007), Science 318(5858): 1917-20; Park, I. H., et al. (2008), Nature 451(7175): 141-6). In brief, introduction of the iPS cell into a non-human preimplantation embryo, like a morula or a blastocyst, is preferably effected by microinjection into a morula or blastocyst or by aggregation of iPS cells with 8-cell or morula embryos. Said chimaeric embryo is then transferred into the uterus of a pseudopregnant non-human female where it develops into an embryo that is finally born.

Generating a transgenic non-human animal line from iPS cells is based on the pluripotence of said iPS cells (i. e., their ability, once injected into a host developing embryo, such as a blastocyst or morula, to participate in embryogenesis and contribute to the germ cells of the resulting animal). As outlined above, the blastocysts containing the injected iPS cells are allowed to develop in the uteri of pseudopregnant non-human females and are born as chimeras. The resultant transgenic non-human animals are chimeric for cells originating from iPS cells and are backcrossed to wildtype non-human animals and screened for animals carrying only the genetic content of an iPS cell so as to identify transgenic animals homozygous for the combination of DNA segments.

The transgenic non-human animals may, for example, be transgenic mice, rats, hamsters, dogs, monkeys, rabbits, pigs, or cows. Preferably, said transgenic non-human animal is a mouse.

Accordingly, the invention also relates to a transgenic non-human animal obtainable by the method of the invention.

Further, the invention relates to a composition comprising an iPS cell obtained by the method of the invention for gene therapy, regenerative medicine, cell therapy or drug screening.

A composition as used herein relates to a composition that comprises iPS cells and preferably further constituents that maintain cell viability of said cell. Such constituents are well-known to the skilled person and comprise, for example, cell media constituents. Further, depending on the intended application the composition may comprise additional constituents, for example, constituents facilitating administration to a patient.

A composition comprising the iPS cells of the invention can be used in a variety of experimental as well as therapeutic scenarios. The iPS cell of the invention being free of transgenic expression elements and containing an unmodified set of endogenous genes are expected to be beneficial in gene therapy, regenerative medicine, cell therapy or drug screening.

Gene therapy, which is based on introducing therapeutic DNA constructs for correcting a genetic defect into germ line cells by ex *vivo* or *in vivo* techniques is one of the most important applications of gene transfer. Suitable vectors and methods for *in vitro* or *in vivo* gene therapy are described in the literature and are known to the person skilled in the art (Davis PB, Cooper MJ., AAPS J. (2007), 19;9(1):E11-7; Li S, Ma Z., Curr Gene Ther. (2001),1(2):201-26). In accordance with the invention, cells obtained from a patient could, for example, be genetically corrected by methods known in the art and described above and subsequently be reprogrammed into iPS cells having the pheno- and genotype of ES cells, by the method of the invention. This evidences the applicability of iPS cells in gene therapy and/or cell therapy.
Regenerative medicine can be used to potentially cure any disease that results from malfunctioning, damaged or failing tissue by either regenerating the damaged tissues in vivo or by growing the tissues and organs in vitro and subsequently implanting them into the patient. The iPS cells of the invention being capable of differentiating into virtually any tissue (ectoderm, mesoderm, endoderm cells) can be used in any aspect of regenerative medicine and hence drastically reduce the need for ES cells.
The iPS cells of the invention can also be used to identify drug targets and test potential therapeutics hence reducing the need for ES cells and in vivo studies. Experimental setups and methods to identify and/or assess effects of a potential drug including, for example, target-site and -specificity, toxicity, bioavailability, are well-known to the person skilled in the art.
Further, the iPS cells may be used to study the prevention and treatment of birth defects or study cell differentiation.

Finally, the present invention relates to the use of the DNA molecule of the invention, the vector of the invention, the method for assembly of a vector of the invention, the somatic cell of the invention, the method of generating an induced pluripotent stem cell of the invention, the induced pluripotent stem cell of the invention, the kit of the invention, the cell line or cell culture collection of the invention, the method to generate a transgenic non-human animal of the invention, the transgenic non-human animal the invention or the composition of the invention as a research tool.

A research tool relates to a means to perform research. Any of the embodiments described herein may be employed by the skilled person in an experimental setting. Said experimental setting varies depending on the object to be achieved by the experiment. The skilled person is in the position to design and conduct experiments incorporating embodiments of the invention in a suitable and ultimately meaningful manner. The embodiments may be used either alone or in connection with each other in an experiment. Generally and as outlined herein above, the present invention is particularly useful when excersised in an experimental setting that would usually require the use of embryonic stem cells such as, e.g., cellular assays, screening assays or re-differentiation studies.
The DNA molecule or the vector of the invention may be useful, for example, in studying the effects of different reprogramming factors on cellular dedifferentiation in different cells. The method of assembly provides the skilled person with the tool to design, prepare and subsequently to use a vector that is uniquely adapted to the specific need of the researcher.
Somatic cells comprising the DNA molecule or the vector of the invention may be used, for example, to study a variety of aspects related to dedifferentiation such as, e.g., spatiotemporal shifts in the expression pattern of genes or of methylation patterns, or the morphological changes leading to changes in aggregation behaviour. Said aspects may, inter alia, also be studied in the resulting iPS cells which have been prepared according to the method of the invention. Said iPS cells can further be subject to studies relating to, e.g., gene therapy, gene targeting, differentiation studies, tests for safety and efficacy of drugs, transplantation of autologous or allogeneic regenerated tissue, tissue repair (e.g., nervous system, heart muscle), diseases like, e.g., Parkinson's disease, heart attack, diabetes, cancer, leukemia or spinal cord injury, embryonal gene expression, genetic manipulation of embryonal genes, early embryology and fetal development, identification of embryonic cell markers, cell migration or apoptosis.
The cell line, the cell culture collection or the kit of the invention are thus also the basic means to enable the skilled person to perform a variety of experiments such as, e.g., outlined herein above, and successfully conduct research.

The figures show:

### Figure 1:

Figure 1 shows the integration of a regulated reprogramming vector into mouse or human dermal fibroblasts by phiC31 Integrase, reprogramming into iPS cells and vector excision from iPS cells through Cre recombinase. The doxycycline inducible expression cassette contains a bidirectional minimal promoter (Pₜₑₜ), activated by the constitutively expressed rtTA activator and silenced by the tTS repressor protein. In the presence of doxycycline two bicistronic mRNAs are initiated at the Pₜₑₜ promoter that lead to the production of the reprogramming proteins OCT4, SOX2, KLF4 and a RFP-MYC fusion protein. The vector is integrated into genomic pseudo attP sites *(*ψ*attP)* of fibroblasts through C31 Int mediated recombination via its single *attB* site. Transfected cells can be selected by the *Neo* resistance gene (pgk-neo). Upon the isolation of induced pluripotent stem (iPS) cell lines the vectors expression cassette can be deleted from the genome by Cre mediated recombination between the flanking *loxP* sites. Only a single 34 bp loxP and a 53 bp *attB*/*P hybrid* site remain within the integration site.

### Figure 2:

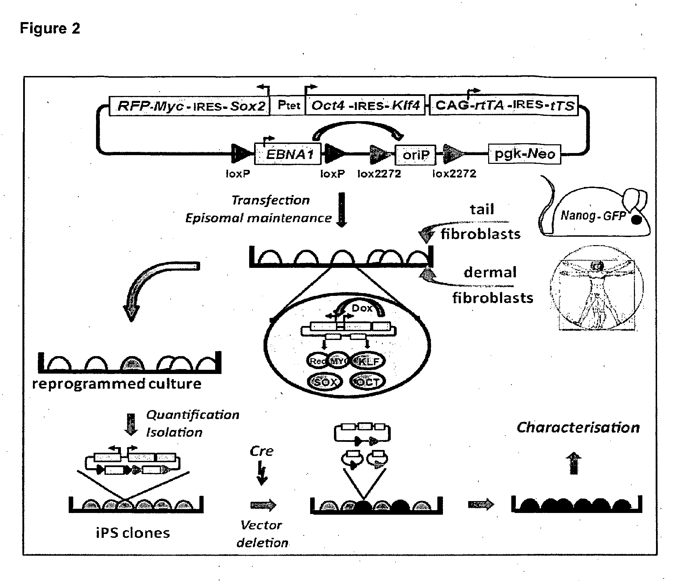

Figure 2 shows the introduction of an episomal, regulated reprogramming vector into mouse or human dermal fibroblasts, reprogramming into iPS cells and vector excision from iPS cells through Cre recombinase. The doxycycline inducible expression cassette contains a bidirectional minimal promoter (Pₜₑₜ), activated by the constitutively expressed rtTA activator and silenced by the tTS repressor protein. In the presence of doxycycline two bicistronic mRNAs are initiated at the Pₜₑₜ promoter that lead to the production of the reprogramming proteins OCT4, SOX2, KLF4 and a RFP-MYC fusion protein. The vector is transfected into fibroblasts and maintained in an episomal state by the action of the EBNA1 protein on the *oriP* element, derived from Epstein Barr virus (Bornkamm, G. W., (2005). Nucleic Acids Res 33(16): e137; Satoh, E., et al. (1997). Biochem Biophys Res Commun 238(3): 795-9; Sclimenti, C. R., et al. (1998). Curr Opin Biotechnol 9(5): 476-9). Transfected cells can be selected by the *Neo* resistance gene (pgk-neo). Upon the isolation of induced pluripotent stem (iPS) cell lines the episomal vector is fragmented into three DNA circles by Cre mediated excision of the *loxP* flanked *EBNA1* gene and the lo*x2272* flanked oriP element which are lost during cell divisions.

### Figure 3:

Figure 3 shows the integration of a regulated reprogramming vector into mouse or human dermal fibroblasts by nuclease stimulated transgene insertion, reprogramming into iPS cells and vector excision from iPS cells through Cre recombinase. The doxycycline inducible expression cassette contains a bidirectional minimal promoter (Pₜₑₜ), activated by the constitutively expressed rtTA activator and silenced by the tTS repressor protein. In the presence of doxycycline two bicistronic mRNAs are initiated at the Pₜₑₜ promoter that lead to the production of the reprogramming proteins OCT4, SOX2, KLF4 and a RFP-MYC fusion protein. The vector becomes integrated into a preselected genomic acceptor site of dermal fibroblasts by cotransfection with an expression vector for a Fok1 nuclease/Zinc finger fusion protein (ZNF-Fok1) that creates a double strand at the specific binding site of the Zinc finger domain (Moehle, E. A., et al. (2007). Proc Natl Acad Sci U S A 104(9): 3055-60; Urnov, F. D., et al. (2005). Nature 435(7042): 646-51). The doxycycline inducible expression cassette, further linked to a neomycin resistance gene (pgk-neo), is flanked by two genomic homology regions (HR1, HR2) located upstream and downstream of the ZNF-Fok1 cutting site. The induced double strand break activates an endogeneous recombination and repair mechanism that leads to the precise integration of the doxycycline inducible expression cassette, flanked by two loxP sites. The genomic integration site is selected such that the function of endogenous genes is not disturbed, e.g. at an intergenic location. Transfected cells can be selected by the *Neo* resistance gene. Upon the isolation of induced pluripotent stem (iPS) cell lines the vectors expression/resistance cassette can be deleted from the genome by Cre mediated recombination between the flanking *loxP* sites. Only a single 34 bp loxP remains within the integration site.

### Figure 4:

Figure 4 schematically shows the integration of a DNA sequence into a target DNA molecule via homology-directed repair (HDR) at a site of induced double strand break as induced, for example, by the action of a ZNF-nuclease outlined in Example 3 and Figure 3.

The examples illustrate the invention:

### Example 1:

### Generation of vector-free iPS cells by targeted integration and excision of a reprogramming vector using site-specific recombinases

For this approach a single reprogramming vector copy is inserted into a defined genomic location of human or mouse dermal fibroblasts by the use of phiC31 Integrase (Fig. 1). The basic vector cassette contains a doxycycline inducible, polycistronic expression cassette that allows to adjust the levels of reprogramming factors Oct4, Sox2, Klf4, and cMyc. By inclusion of a C31 Integrase attB site the vector is integrated into a limited number of genomic pseudo attB sites in the human or mouse genome, many of them occur in intergenic regions (Calos, M. P. (2006). Curr Gene Ther 6(6): 633-45; Chalberg, T. W., H. L. Genise, et al. (2005). Invest Ophthalmol Vis Sci 46(6): 2140-6; Chalberg, T. W., et al. (2006 J Mol Biol 357(1): 28-48; Ortiz-Urda, S., B. Thyagarajan, et al. (2002). Nat Med 8(10): 1166-70; Thyagarajan, B., E. C. Olivares, et al. (2001). Mol Cell Biol 21 (12): 3926-34); for its removal two loxP sites are included. Upon the establishment of iPS cell lines and characterisation of the genomic integration site, the vector copy is removed from the genome of selected iPS cell lines through Cre recombinase mediated deletion (Branda, C. S., et al. (2004). Dev Cell 6(1): 7-28).
Surprisingly, it was found that the expression of reprogramming factors from the single vector copy located at an intergenic region upon integration into a genomic pseudo attP site through C31 integrase, is able induce the dedifferentiation of fibroblasts into the ES cell-like phenotype of iPS cells. The vector-free iPS cell lines are characterised by analysis for stem cell marker expression (ALP, OCT4, NANOG, SOX2, SSEA3/4), gene expression analysis through microarrays and the assessment of differentiation capacity and karyotyping. The pluripotency of murine iPS cell lines is tested by the production of germline chimaeric mice through blastocyst injection while human iPS cell lines are analysed through *in vitro* differentiation into neural, glia and cardiac cells. The *in vivo* differentiation capacity of human iPS cell lines is tested by induction of teratoma formation in immunodeficient SCID mice.

### Vector construction and cell culture

The ∼12 kb vector cassette is assembled within pBluescript as plasmid vector using the doxycycline responsive cassette from pRTS1 (Bornkamm, G. W., et al. (2005). Nucleic Acids Res 33(16): e137) and the mouse and human cDNA coding regions of *Oct4, Sox2, KLF4* and *c-Myc* (SEQ ID No.: 1-4, 7-10) amplified from IMAGE cDNA clones (imaGenes, Berlin), and connected via an *EMCV* derived internal ribosomal entry site (IRES) (Hellen, C. U., et al. (1995). Curr Top Microbiol Immunol 203: 31-63) or the *Thosea* derived 2a self-cleaving peptide sequence (Osborn, M. J., et al. (2005). Mol Ther 12(3): 569-74). The *c-Myc* gene is fused with TagRFP red fluorescent protein (Merzlyak, E. M., et al. (2007). Nat Methods 4(7): 555-7) as described for GFP (Yin, X., et al. (2001). Oncogene 20(34): 4650-64). The mouse and human REPRO cassette is inserted into a backbone containing a Neo gene and an attB recognition site for C31 Integrase (Thyagarajan, B., et al. (2001). Mol Cell Biol 21(12): 3926-34), flanked by two Cre recombinase (loxP) recognition sites (Siegel, R. W., et al. (2001 FEBS Lett 499(1-2): 147-53). This vector is introduced by into tail tip fibroblasts isolated from OCT4-GFP transgenic mice (Yoshimizu, T., et al. (1999). Dev Growth Differ 41(6): 675-84) and into normal human adult dermal fibroblasts (PromoCell GmbH, Heidelberg) together with the CAG-C31Int-bpA expression vectors for C31 Integrase (Hitz, C., et al. (2007). Nucleic Acids Res 35(12): e90) by electroporation or lipofection. Cultures are initially kept with 0.5 ug/ml doxycycline for maximal activation of gene expression. At day 2 the quantification of RFP positive cells allows to estimate the rate of transient transfection. Next, transfected cells are selected with G418 and transferred 2 days later on mitotic inactivated embryonic feeder cells from Neo transgenic mice in murine or human ES cell medium. From day 7 on G418 is omitted from the cultures and the doxycycline concentration varied between 1-500 ng/ml culture medium. The transgene expression level is monitored by the quantification of the RFP-Myc fluorescence signal while iPS cells are detected via the OCT-GFP reporter. At day 21-30 GFP positive colonies with ES cell like morphology are isolated and individually expanded. To analyse the genomic integration site genomic DNA of such clones is isolated, digested with restriction enzymes that do not cut within the vector, and recircularised with T4 DNA ligase. Rescued plamids are transformed into competent E. coli cells and the genomic DNA flanking the vector is determined by DNA sequencing using primers located at the ends of the integrated vector. Upon identification of the genomic integration site by comparison with the mouse or human genome sequence selected clones, in which the vector does not disturb the function of an endogenous gene, are transiently transfected with the Cre expression vector pCAGCrebpA (Hitz, C., et al. (2007). Nucleic Acids Res 35(12): e90) and analysed for vector loss by PCR. The pluripotency and integrity of the vector-free murine iPS cell lines is characterised by analysis for stem cell markers (ALP, OCT4, NANOG, SOX2, SSEA3/4), microarray analysis, the assessment of differentiation capacity and karyotyping. To assess pluripotency murine iPS cell lines are used to generate chimaeric mice by injection into blastocysts of differing coat color and glucose phosphate isomerase (GPI) isotype. The extent of chimaerism in the major organs is quantified by GPI analysis while the iPS germline contribution will be tested by mating and coat color analysis. The differentiation capacity of human iPS cell lines is analysed through *in vitro* differentiation into neural, glia and cardiac cells. The *in vivo* differentiation capacity of human iPS cell lines is tested by induction of teratoma formation in immunodeficient SCID mice.

### Example 2:

### Generation of vector-free iPS cells using a self-replicating, episomal reprogramming vector and its removal through site-specific recombination.

For this approach the reprogramming expression cassette is combined with genetic elements that enable extrachromosomal (episomal) vector replication at low copy number, the EBNA1 and oriP of the Epstein Barr virus (Bornkamm, G. W., (2005). Nucleic Acids Res 33(16): e137; Satoh, E., et al. (1997). Biochem Biophys Res Commun 238(3): 795-9; Sclimenti, C. R., et al. (1998). Curr Opin Biotechnol 9(5): 476-9). The basic vector cassette contains a doxycycline inducible, polycistronic expression cassette that allows to adjust the levels of the reprogramming factors Oct4, Sox2, Klf4, and cMyc. By inclusion of wildtype and mutant loxP sites flanking the EBNA1 and oriP elements the vector can be disintegrated through Cre mediated recombination (Fig.2) (Branda, C. S., et al. (2004). Dev Cell 6(1): 7-28). Upon the establishment of iPS cell lines from fibroblasts, the episomal vector copies are disrupted through Cre mediated recombination. The vector segments are gradually lost over cell division and vector-free subclones, identified by PCR and Southern blot analysis of genomic DNA, are isolated and expanded.
Surprisingly, it was found that expression of reprogramming factors from a low copy episomal vector, is able induce the dedifferentiation of fibroblasts into the ES cell-like phenotype of iPS cells and that the episomal vector can be removed from these cells by use of a site-specific recombinase. The vector-free iPS cell lines are characterised by analysis for stem cell marker expression (ALP, OCT4, NANOG, SOX2, SSEA3/4), gene expression analysis through microarrays and the assessment of differentiation capacity and karyotyping. The pluripotency of murine iPS cell lines is tested by the production of germline chimaeric mice through blastocyst injection while human iPS cell lines are analysed through *in vitro* differentiation into neural, glia and cardiac cells. The *in vivo* differentiation capacity of human iPS cell lines is tested by induction of teratoma formation in immunodeficient SCID mice.

### Vector construction and cell culture

The ∼12 kb vector cassette is assembled within pBluescript as plasmid vector using the doxycycline responsive cassette from pRTS1 (Bornkamm, G. W., et al. (2005). Nucleic Acids Res 33(16): e137) and the mouse and human cDNA coding regions of *Oct4, Sox2, KLF4* and *c-Myc* (SEQ ID No.: 1-4, 7-10) amplified from IMAGE cDNA clones (ImaGenes, Berlin), and connected via an *EMCV* derived internal ribosomal entry site (IRES) (Hellen, C. U., et al. (1995). Curr Top Microbiol Immunol 203: 31-63) or the *Thosea* derived 2a self-cleaving peptide sequence (Osborn, M. J., et al. (2005). Mol Ther 12(3): 569-74). The *c-Myc* gene is fused with TagRFP red fluorescent protein (Merzlyak, E. M., et al. (2007). Nat Methods 4(7): 555-7) as described for GFP (Yin, X., et al. (2001). Oncogene 20(34): 4650-64). The mouse and human REPRO cassette is inserted into a backbone containing a Neo gene and the *EBNA1*/*oriP* element from pRTS1 (Bornkamm, G. W., (2005). Nucleic Acids Res 33(16): e137) flanked with *loxP* or *lox2272* sites (Siegel, R. W., et al. (2001 FEBS Lett 499(1-2): 147-53). This vector is introduced by into tail tip fibroblasts isolated from OCT4-GFP transgenic mice (Yoshimizu, T., et al. (1999). Dev Growth Differ 41 (6): 675-84) and into normal human adult dermal fibroblasts (PromoCell GmbH, Heidelberg by electroporation or lipofection. Cultures are initially kept with 0.5 ug/ml doxycycline for maximal activation of gene expression. At day 2 the quantification of RFP positive cells allows to estimate the rate of transient transfection. Next, transfected cells are selected with G418 and transferred 2 days later on mitotic inactivated embryonic feeder cells from Neo transgenic mice in murine or human ES cell medium. From day 7 on G418 is omitted from the cultures and the doxycycline concentration varied between 1-500 ng/ml culture medium. The transgene expression level is monitored by the quantification of the RFP-Myc fluorescence signal while iPS cells are detected via the OCT-GFP reporter. At day 21-30 GFP positive colonies with ES cell like morphology are isolated and individually expanded. Selected clones are transiently transfected with the Cre expression vector pCAGCrebpA (Hitz, C., et al. (2007). Nucleic Acids Res 35(12): e90) and analysed for vector loss by PCR. The pluripotency and integrity of the vector-free murine iPS cell lines is characterised by analysis for stem cell markers (ALP, OCT4, NANOG, SOX2, SSEA3/4), microarray analysis, the assessment of differentiation capacity and karyotyping. To assess pluripotency murine iPS cell lines will be used to generate chimaeric mice by injection into blastocysts of differing coat color and glucose phosphate isomerase (GPI) isotype. The extent of chimaerism in the major organs is quantified by GPI analysis while the iPS germline contribution will be tested by mating and coat color analysis. The differentiation capacity of human iPS cell lines is analysed through *in vitro* differentiation into neural, glia and cardiac cells. The *in vivo* differentiation capacity of human iPS cell lines is tested by induction of teratoma formation in immunodeficient SCID mice.

### Example 3:

### Generation of vector-free iPS cells using nuclease stimulated transgene insertion and its removal through site-specific recombination.

In this approach the targeted integration of a regulated reprogramming vector into the genome of mouse or human dermal fibroblasts is accomplished by nuclease stimulated transgene insertion. Somatic cells are reprogrammed into iPS cells and the vector excised from iPS cells through Cre recombinase (Fig. 3). The vectors doxycycline inducible expression cassette contains a bidirectional minimal promoter (Pₜₑₜ), activated by the constitutively expressed rtTA activator and silenced by the tTS repressor protein. In the presence of doxycycline two bicistronic mRNAs are initiated at the Pₜₑₜ promoter that lead to the production of the reprogramming proteins OCT4, SOX2, KLF4 and a RFP-MYC fusion protein. The vector becomes integrated into a preselected genomic acceptor site of dermal fibroblasts by cotransfection with an expression vector for a Fok1 nuclease/Zinc finger fusion protein (ZNF-Fok1) that creates a double strand at the specific binding site of the Zinc finger domain (Moehle, E. A., et al. (2007). Proc Natl Acad Sci U S A 104(9): 3055-60; Urnov, F. D., et al. (2005). Nature 435(7042): 646-51). The doxycycline inducible expression cassette, further linked to neomycin resistance gene (pgk-neo), is flanked by two 750 bp genomic homology regions (HR1, HR2) located upstream and downstream of the ZNF-Fok1 cutting site within the first intron of the human Rosa26 locus (Zambrowicz, B. P., A. Imamoto, et al. (1997). Proc Natl Acad Sci U S A 94(8): 3789-94; Irion, S., et al. (2007) Nat Biotechnol 25(12): 1477-82). The induced double strand break activates an endogeneous recombination and repair mechanism that leads to the precise integration of the doxycycline inducible expression cassette, flanked by two loxP sites. Transfected cells are selected by the *Neo* resistance gene. Upon the isolation of induced pluripotent stem (iPS) cell lines the vectors expression/resistance cassette can be deleted from the genome by Cre mediated recombination between the flanking *loxP* sites (Branda, C. S., et al. (2004). Dev Cell 6(1): 7-28). Only a single 34 bp loxP remains within the integration site.
Surprisingly, it was found that expression of reprogramming factors from a single vector copy, is able induce the dedifferentiation of fibroblasts into the ES cell-like phenotype of iPS cells. The vector-free iPS cell lines are characterised by analysis for stem cell marker expression (ALP, OCT4, NANOG, SOX2, SSEA3/4), gene expression analysis through microarrays and the assessment of differentiation capacity and karyotyping. The pluripotency of murine iPS cell lines is tested by the production of germline chimaeric mice through blastocyst injection while human iPS cell lines are analysed through *in vitro* differentiation into neural, glia and cardiac cells. The *in vivo* differentiation capacity of human iPS cell lines is tested by induction of teratoma formation in immunodeficient SCID mice.

### Vector construction and cell culture

The ∼12 kb vector cassette is assembled within pBluescript as plasmid vector using the doxycycline responsive cassette from pRTS1 (Bornkamm, G. W., et al. (2005). Nucleic Acids Res 33(16): e137) and the mouse and human cDNA coding regions of *Oct4, Sox2, KLF4* and *c-Myc* (SEQ ID No.: 1-4., 7-10) amplified from IMAGE cDNA clones (ImaGenes, Berlin), and connected via an *EMCV* derived internal ribosomal entry site (IRES) (Hellen, C. U., et al. (1995). Curr Top Microbiol Immunol 203: 31-63) or the *Thosea* derived 2a self-cleaving peptide sequence (Osborn, M. J., et al. (2005). Mol Ther 12(3): 569-74). The *c-Myc* gene is fused with TagRFP red fluorescent protein (Merzlyak, E. M., et al. (2007). Nat Methods 4(7): 555-7) as described for GFP (Yin, X., et al. (2001). Oncogene 20(34): 4650-64). The mouse and human reprogramming cassette is inserted into a backbone containing a Neo gene, two *loxP* sites and two 750 bp homology regions derived from the human or mouse Rosa26 locus (Zambrowicz, B. P., A. Imamoto, et al. (1997). Proc Natl Acad Sci U S A 94(8): 3789-94; Irion, S., et al. (2007) Nat Biotechnol 25(12): 1477-82). This vector is introduced by into tail tip fibroblasts isolated from OCT4-GFP transgenic mice (Yoshimizu, T., et al. (1999). Dev Growth Differ 41 (6): 675-84) and into normal human adult dermal fibroblasts (PromoCell GmbH, Heidelberg) together with an expression vector for Zinc finger/Fok1 nuclease fusion protein, that specifically binds and cuts the junction between the Rosa26 homology regions, by electroporation or lipofection. Cultures are initially kept with 0.5 ug/ml doxycycline for maximal activation of gene expression. At day 2 the quantification of RFP positive cells allows to estimate the rate of transient transfection. Next, transfected cells are selected with G418 and transferred 2 days later on mitotic inactivated embryonic feeder cells from Neo transgenic mice in murine or human ES cell medium. From day 7 on G418 is omitted from the cultures and the doxycycline concentration varied between 1-500 ng/ml culture medium. The transgene expression level is monitored by the quantification of the RFP-Myc fluorescence signal while iPS cells are detected via the OCT-GFP reporter. At day 21-30 GFP positive colonies with ES cell like morphology are isolated and individually expanded. The targeting of the vector into the Rosa26 locus is confirmed by Southern blotting of genomic DNA using specific hybridisation probes. Selected clones are transiently transfected with the Cre expression vector pCAGCrebpA (Hitz, C., et al. (2007). Nucleic Acids Res 35(12): e90) and analysed for vector loss by PCR. The pluripotency and integrity of the vector-free murine iPS cell lines is characterised by analysis for stem cell markers (ALP, OCT4, NANOG, SOX2, SSEA3/4), microarray analysis, the assessment of differentiation capacity and karyotyping. To assess pluripotency murine iPS cell lines will be used to generate chimaeric mice by injection into blastocysts of differing coat color and glucose phosphate isomerase (GPI) isotype. The extent of chimaerism in the major organs is quantified by GPI analysis while the iPS germline contribution will be tested by mating and coat color analysis. The differentiation capacity of human iPS cell lines is analysed through *in vitro* differentiation into neural, glia and cardiac cells. The *in vivo* differentiation capacity of human iPS cell lines is tested by induction of teratoma formation in immunodeficient SCID mice.

## Claims

1. A DNA molecule comprising:
(a) a first DNA sequence comprising:
(aa) a coding sequence giving rise upon transcription to a factor that contributes to the reprogramming of a somatic cell into an induced pluripotent stem (iPS) cell;
(ab) a promoter mediating the transcription of said coding sequence; and
(ac) two sequence motifs that mediate excision of (aa) and/or (ab) from the DNA molecule, wherein one sequence motif is positioned 5' and the other sequence motif is positioned 3' of the sequence to be excised;
(b) a second DNA sequence comprising a sequence motif that mediates site-specific integration of (a) into another DNA molecule.

2. A DNA molecule comprising:
(a) a first DNA sequence comprising:
(aa) a coding sequence giving rise upon transcription to a factor that contributes to the reprogramming of a somatic cell into an induced pluripotent stem (iPS) cell; and
(ab) a promoter mediating the transcription of said coding sequence;
(b) a second DNA sequence comprising:
(ba) a sequence motif that mediates extrachromosomal self-replication of the DNA-molecule; and
(bb) two sequence motifs that mediate excision of at least said sequence motif of (ba) from the DNA molecule, wherein one sequence motif is located 5' of (ba) and the other sequence motif 3' of (ba).

3. The DNA molecule of claim 1 or 2, wherein said coding sequence of (aa) is selected from the group consisting of Oct, Sox, Klf, Myc, Nanog and Lin coding sequences.

4. The DNA molecule of any one of claims 1 to 3, wherein said coding sequence of (aa) is selected from the group consisting of Oct3/4, Sox1, Sox2, Sox3, Sox15, Sox18, Klf1, Klf2, Klf4, Klf5, n-Myc, I-Myc, c-Myc, Nanog and Lin28 coding sequences.

5. The DNA molecule of any one of claims 1 to 4, wherein said coding sequence of (aa) comprises 3 coding sequences selected from the combinations of Oct3/4, a Sox and a Klf coding sequence.

6. The DNA molecule of claim 5, wherein said coding sequence of (aa) comprises the coding sequences of Oct3/4, Sox2 and Klf4.

7. The DNA molecule of any one of claims 1 to 4, wherein said coding sequence of (aa) comprises 4 coding sequences selected from the combinations of Oct3/4, a Sox, a Klf, and a Myc coding sequence and Oct3/4, a Sox coding sequence, Nanog and Lin28.

8. The DNA molecule of claim 7, wherein said coding sequence of (aa) comprises 4 coding sequences selected from the combinations of Oct3/4, Sox2, Klf4, c-Myc; and Oct3/4, Sox2, Nanog, Lin28.

9. The DNA molecule of any one of claims 1 to 8, wherein the promoter (ab) is an inducible promoter.

10. The DNA molecule of claim 9, wherein the promoter (ab) is inducible by doxycycline.

11. The DNA molecule of any one of claims 1 to 10, wherein the promoter (ab) is a bidirectional minimal promoter.

12. The DNA molecule of any one of claims 1, and 3 to 11, wherein the sequence motif of the second DNA sequence (b) is selected from the group consisting of attB, attP and an ITR (inverted terminal repeat), wherein the ITR is recognized by an adeno-associated virus (AAV) integrase.

13. The DNA molecule of any one of claims 1, and 3 to 11 wherein the sequence motif of the second DNA sequence (b) comprises two sequences that flank the first DNA sequence (a) and are as a combined sequence essentially identical to a sequence at the site of integration.

14. The DNA molecule of any one of claims 1 to 14, wherein the sequence motifs allowing excision of (aa) and/or (ab) of claim 1 or said sequence motif (ba) of claim 2 are lox sequences.

15. The DNA molecule of any one of claims 2 to 11, wherein the sequence motif (ba) comprises an EBNA1 and an oriP element.

16. The DNA molecule of claim 15, wherein said EBNA1 is flanked by a first type of lox sequences and the oriP element is flanked by a second type of lox sequences and wherein
(a) said first type of lox sequences are recognized and recombined by a Cre-recombinase but not recombined with said second type of lox sequences; and
(b) said second type of lox sequences are recognized and recombined by the recombinase of (a) but not recombined with said first type of lox sequences.

17. A vector comprising the DNA molecule of any one of claims 1, and 3 to 14.

18. A vector comprising the DNA molecule of any one of claims 2 to 11, and 15 or 16.

19. A method for assembly of a vector of claim 17 or 18 comprising the step of
(I) integrating:
(Ia) a sequence comprising the sequences (aa), (ab), (ac) and (b) as mentioned in claim 1; or
(Ib) a sequence comprising the sequences (aa), (ab), (ba) and (bb) as mentioned in claim 2,
either individually or combined as a contiguous sequence into a vector sequence; or
(II) circularizing a contiguous sequence comprising the sequences of (Ia) or (Ib).

20. A somatic cell comprising the DNA molecule of any one of claims 1 to 16 or the vector of claim 17 or 18.

21. A method to generate an induced pluripotent stem (iPS) cell comprising the steps of:
(i) introducing the DNA molecule of any one of claims 1, and 3 to 14 or the vector of claim 17 into a somatic cell;
(ii) allowing the DNA molecule or the vector of step (i) to integrate into the genomic DNA of said somatic cell; and
(iii) excising the sequence that is enclosed by the two sequence motifs of (ac) from the DNA molecule,
wherein step (iii) is performed after reprogramming of said somatic cell has taken place.

22. A method to generate an induced pluripotent stem cell comprising the steps of:
(i) introducing the DNA molecule of any one of claims 2 to 11, and 15 or 16 or the vector of claim 18 into a somatic cell; and
(ii) excising the sequence motif (ba) from the DNA molecule,
wherein step (ii) is performed after reprogramming of said somatic cell has taken place.

23. An induced pluripotent stem cell obtainable by the method of claims 21 or 22.

24. A kit comprising the DNA molecule of any one of claims 1 to 16, the sequences (aa), (ab), (ac) and (b) as mentioned in claim 1, the sequences (aa), (ab), (ba) and (bb) as mentioned in claim 2, the vector of claim 17 or 18, or the induced pluripotent cell of claim 23.

25. A cell line or cell culture collection comprising the induced pluripotent stem cell of claim 23.

26. A method to generate a transgenic non-human animal comprising the steps of claims 21 or 22 and the further steps of:
(i) introducing the induced pluripotent stem cells into a non-human blastocyst;
(ii) transferring the blastocyst into the uterus of a female non-human animal; and
(iii) allowing the blastocyst to develop into an embryo.

27. A transgenic non-human animal obtainable by the method of claim 26.

28. Composition comprising an iPS cell obtained by the method of claims 21 or 22 for gene therapy, regenerative medicine, cell therapy or drug screening.

29. Use of the DNA molecule of any one of claims 1 to 16, the vector of claim 17 or 18, the method for assembly of a vector of claim 19, the somatic cell of claim 20, the method of generating an induced pluripotent stem cell of claim 21 or 22, the induced pluripotent stem cell of claim 23, the kit of claim 24, the cell line or cell culture collection of claim 25, the method to generate a transgenic non-human animal of claim 26, the transgenic non-human animal of claim 27 or the composition of claim 28 as a research tool.


SEQUENCE LISTING

<110>  Helmholtz Zentrum München - Deutsches Forschungszentrum für Gesundheit
und Umwelt (GmbH)

<120>  Vectors and methods for generating vector-free induced
       pluripotent stem (iPS) cells using site-specific recombination

<130>  P1575 EP

<160>  25

<170>  PatentIn version 3.3

<210>  1
<211>  1059
<212>  DNA
<213>  mus musculus

<400>  1
atggctggac acctggcttc agacttcgcc ttctcacccc caccaggtgg gggtgatggg      60

tcagcagggc tggagccggg ctgggtggat cctcgaacct ggctaagctt ccaagggcct     120

ccaggtgggc ctggaatcgg accaggctca gaggtattgg ggatctcccc atgtccgccc     180

gcatacgagt tctgcggagg gatggcatac tgtggacctc aggttggact gggcctagtc     240

ccccaagttg gcgtggagac tttgcagcct gagggccagg caggagcacg agtggaaagc     300

aactcagagg gaacctcctc tgagccctgt gccgaccgcc ccaatgccgt gaagttggag     360

aaggtggaac caactcccga ggagtcccag gacatgaaag ccctgcagaa ggagctagaa     420

cagtttgcca agctgctgaa gcagaagagg atcaccttgg ggtacaccca ggccgacgtg     480

gggctcaccc tgggcgttct ctttggaaag gtgttcagcc agaccaccat ctgtcgcttc     540

gaggccttgc agctcagcct taagaacatg tgtaagctgc ggcccctgct ggagaagtgg     600

gtggaggaag ccgacaacaa tgagaacctt caggagatat gcaaatcgga gaccctggtg     660

caggcccgga agagaaagcg aactagcatt gagaaccgtg tgaggtggag tctggagacc     720

atgtttctga agtgcccgaa gccctcccta cagcagatca ctcacatcgc caatcagctt     780

gggctagaga aggatgtggt tcgagtatgg ttctgtaacc ggcgccagaa gggcaaaaga     840

tcaagtattg agtattccca acgagaagag tatgaggcta cagggacacc tttcccaggg     900

ggggctgtat cctttcctct gcccccaggt ccccactttg gcaccccagg ctatggaagc     960

ccccacttca ccacactcta ctcagtccct tttcctgagg gcgaggcctt tccctctgtt    1020

cccgtcactg ctctgggctc tcccatgcat tcaaactga                            1059

<210>  2
<211>  352
<212>  PRT
<213>  mus musculus

<400>  2

Met Ala Gly His Leu Ala Ser Asp Phe Ala Phe Ser Pro Pro Pro Gly
1               5                   10                  15

Gly Gly Asp Gly Ser Ala Gly Leu Glu Pro Gly Trp Val Asp Pro Arg
            20                  25                  30

Thr Trp Leu Ser Phe Gln Gly Pro Pro Gly Gly Pro Gly Ile Gly Pro
        35                  40                  45

Gly Ser Glu Val Leu Gly Ile Ser Pro Cys Pro Pro Ala Tyr Glu Phe
    50                  55                  60

Cys Gly Gly Met Ala Tyr Cys Gly Pro Gln Val Gly Leu Gly Leu Val
65                  70                  75                  80

Pro Gln Val Gly Val Glu Thr Leu Gln Pro Glu Gly Gln Ala Gly Ala
                85                  90                  95

Arg Val Glu Ser Asn Ser Glu Gly Thr Ser Ser Glu Pro Cys Ala Asp
            100                 105                 110

Arg Pro Asn Ala Val Lys Leu Glu Lys Val Glu Pro Thr Pro Glu Glu
        115                 120                 125

Ser Gln Asp Met Lys Ala Leu Gln Lys Glu Leu Glu Gln Phe Ala Lys
    130                 135                 140

Leu Leu Lys Gln Lys Arg Ile Thr Leu Gly Tyr Thr Gln Ala Asp Val
145                 150                 155                 160

Gly Leu Thr Leu Gly Val Leu Phe Gly Lys Val Phe Ser Gln Thr Thr
                165                 170                 175

Ile Cys Arg Phe Glu Ala Leu Gln Leu Ser Leu Lys Asn Met Cys Lys
            180                 185                 190

Leu Arg Pro Leu Leu Glu Lys Trp Val Glu Glu Ala Asp Asn Asn Glu
        195                 200                 205

Asn Leu Gln Glu Ile Cys Lys Ser Glu Thr Leu Val Gln Ala Arg Lys
    210                 215                 220

Arg Lys Arg Thr Ser Ile Glu Asn Arg Val Arg Trp Ser Leu Glu Thr
225                 230                 235                 240

Met Phe Leu Lys Cys Pro Lys Pro Ser Leu Gln Gln Ile Thr His Ile
                245                 250                 255

Ala Asn Gln Leu Gly Leu Glu Lys Asp Val Val Arg Val Trp Phe Cys
            260                 265                 270

Asn Arg Arg Gln Lys Gly Lys Arg Ser Ser Ile Glu Tyr Ser Gln Arg
        275                 280                 285

Glu Glu Tyr Glu Ala Thr Gly Thr Pro Phe Pro Gly Gly Ala Val Ser
    290                 295                 300

Phe Pro Leu Pro Pro Gly Pro His Phe Gly Thr Pro Gly Tyr Gly Ser
305                 310                 315                 320

Pro His Phe Thr Thr Leu Tyr Ser Val Pro Phe Pro Glu Gly Glu Ala
                325                 330                 335

Phe Pro Ser Val Pro Val Thr Ala Leu Gly Ser Pro Met His Ser Asn
            340                 345                 350

<210>  3
<211>  1083
<212>  DNA
<213>  homo sapiens

<400>  3
atggcgggac acctggcttc ggatttcgcc ttctcgcccc ctccaggtgg tggaggtgat      60

gggccagggg ggccggagcc gggctgggtt gatcctcgga cctggctaag cttccaaggc     120

cctcctggag ggccaggaat cgggccgggg gttgggccag gctctgaggt gtgggggatt     180

cccccatgcc ccccgccgta tgagttctgt gggggggatgg cgtactgtgg gccccaggtt     240

ggagtggggc tagtgcccca aggcggcttg gagacctctc agcctgaggg cgaagcagga     300

gtcggggtgg agagcaactc cgatggggcc tccccggagc cctgcaccgt cacccctggt     360

gccgtgaagc tggagaagga gaagctggag caaaacccgg aggagtccca ggacatcaaa     420

gctctgcaga aagaactcga gcaatttgcc aagctcctga agcagaagag gatcaccctg     480

ggatatacac aggccgatgt ggggctcacc ctgggggttc tatttgggaa ggtattcagc     540

caaacgacca tctgccgctt tgaggctctg cagcttagct tcaagaacat gtgtaagctg     600

cggcccttgc tgcagaagtg ggtggaggaa gctgacaaca atgaaaatct tcaggagata     660

tgcaaagcag aaaccctcgt gcaggcccga aagagaaagc gaaccagtat cgagaaccga     720

gtgagaggca acctggagaa tttgttcctg cagtgcccga aacccacact gcagcagatc     780

agccacatcg cccagcagct tgggctcgag aaggatgtgg tccgagtgtg gttctgtaac     840

cggcgccaga agggcaagcg atcaagcagc gactatgcac aacgagagga ttttgaggct     900

gctgggtctc ctttctcagg gggaccagtg tcctttcctc tggccccagg gccccatttt     960

ggtaccccag gctatgggag ccctcacttc actgcactgt actcctcggt ccctttccct    1020

gaggggggaag cctttccccc tgtctccgtc accactctgg gctctcccat gcattcaaac 1080

tga 1083

<210> 4
<211> 360
<212> PRT
<213> homo sapiens

<400> 4

Met Ala Gly His Leu Ala Ser Asp Phe Ala Phe Ser Pro Pro Pro Gly
1               5                   10                  15

Gly Gly Gly Asp Gly Pro Gly Gly Pro Glu Pro Gly Trp Val Asp Pro
            20                  25                  30

Arg Thr Trp Leu Ser Phe Gln Gly Pro Pro Gly Gly Pro Gly Ile Gly
        35                  40                  45

Pro Gly Val Gly Pro Gly Ser Glu Val Trp Gly Ile Pro Pro Cys Pro
    50                  55                  60

Pro Pro Tyr Glu Phe Cys Gly Gly Met Ala Tyr Cys Gly Pro Gln Val
65                  70                  75                  80

Gly Val Gly Leu Val Pro Gln Gly Gly Leu Glu Thr Ser Gln Pro Glu
                85                  90                  95

Gly Glu Ala Gly Val Gly Val Glu Ser Asn Ser Asp Gly Ala Ser Pro
            100                 105                 110

Glu Pro Cys Thr Val Thr Pro Gly Ala Val Lys Leu Glu Lys Glu Lys
        115                 120                 125

Leu Glu Gln Asn Pro Glu Glu Ser Gln Asp Ile Lys Ala Leu Gln Lys
    130                 135                 140

Glu Leu Glu Gln Phe Ala Lys Leu Leu Lys Gln Lys Arg Ile Thr Leu
145                 150                 155                 160

Gly Tyr Thr Gln Ala Asp Val Gly Leu Thr Leu Gly Val Leu Phe Gly
                165                 170                 175

Lys Val Phe Ser Gln Thr Thr Ile Cys Arg Phe Glu Ala Leu Gln Leu
            180                 185                 190

Ser Phe Lys Asn Met Cys Lys Leu Arg Pro Leu Leu Gln Lys Trp Val
        195                 200                 205

Glu Glu Ala Asp Asn Asn Glu Asn Leu Gln Glu Ile Cys Lys Ala Glu
    210                 215                 220

Thr Leu Val Gln Ala Arg Lys Arg Lys Arg Thr Ser Ile Glu Asn Arg
225                 230                 235                 240

Val Arg Gly Asn Leu Glu Asn Leu Phe Leu Gln Cys Pro Lys Pro Thr
                245                 250                 255

Leu Gln Gln Ile Ser His Ile Ala Gln Gln Leu Gly Leu Glu Lys Asp
            260                 265                 270

Val Val Arg Val Trp Phe Cys Asn Arg Arg Gln Lys Gly Lys Arg Ser
        275                 280                 285

Ser Ser Asp Tyr Ala Gln Arg Glu Asp Phe Glu Ala Ala Gly Ser Pro
    290                 295                 300

Phe Ser Gly Gly Pro Val Ser Phe Pro Leu Ala Pro Gly Pro His Phe
305                 310                 315                 320

Gly Thr Pro Gly Tyr Gly Ser Pro His Phe Thr Ala Leu Tyr Ser Ser
                325                 330                 335

Val Pro Phe Pro Glu Gly Glu Ala Phe Pro Pro Val Ser Val Thr Thr
            340                 345                 350

Leu Gly Ser Pro Met His Ser Asn
        355                 360

<210>  5
<211>  960
<212>  DNA
<213>  mus musculus

<400>  5
atgtataaca tgatggagac ggagctgaag ccgccgggcc cgcagcaagc ttcggggggc      60

ggcggcggag gaggcaacgc cacggcggcg gcgaccggcg gcaaccagaa gaacagcccg     120

gaccgcgtca agaggcccat gaacgccttc atggtatggt cccgggggca gcggcgtaag     180

atggcccagg agaaccccaa gatgcacaac tcggagatca gcaagcgcct gggcgcggag     240

tggaaacttt tgtccgagac cgagaagcgg ccgttcatcg acgaggccaa gcggctgcgc     300

gctctgcaca tgaaggagca cccggattat aaataccggc cgcggcggaa aaccaagacg     360

ctcatgaaga aggataagta cacgcttccc ggaggcttgc tggcccccgg cgggaacagc     420

atggcgagcg gggttggggt gggcgccggc ctgggtgcgg gcgtgaacca gcgcatggac     480

agctacgcgc acatgaacgg ctggagcaac ggcagctaca gcatgatgca ggagcagctg     540

ggctacccgc agcacccggg cctcaacgct cacggcgcgg cacagatgca accgatgcac 600

cgctacgacg tcagcgccct gcagtacaac tccatgacca gctcgcagac ctacatgaac 660

ggctcgccca cctacagcat gtcctactcg cagcagggca cccccggtat ggcgctgggc 720

tccatgggct ctgtggtcaa gtccgaggcc agctccagcc cccccgtggt tacctcttcc 780

tcccactcca gggcgccctg ccaggccggg gacctccggg acatgatcag catgtacctc 840

cccggcgccg aggtgccgga gcccgctgcg cccagtagac tgcacatggc ccagcactac 900

cagagcggcc cggtgcccgg cacggccatt aacggcacac tgcccctgtc gcacatgtga 960

<210> 6
<211> 319
<212> PRT
<213> mus musculus

<400> 6

Met Tyr Asn Met Met Glu Thr Glu Leu Lys Pro Pro Gly Pro Gln Gln
1               5                   10                  15

Ala Ser Gly Gly Gly Gly Gly Gly Gly Asn Ala Thr Ala Ala Ala Thr
            20                  25                  30

Gly Gly Asn Gln Lys Asn Ser Pro Asp Arg Val Lys Arg Pro Met Asn
        35                  40                  45

Ala Phe Met Val Trp Ser Arg Gly Gln Arg Arg Lys Met Ala Gln Glu
    50                  55                  60

Asn Pro Lys Met His Asn Ser Glu Ile Ser Lys Arg Leu Gly Ala Glu
65                  70                  75                  80

Trp Lys Leu Leu Ser Glu Thr Glu Lys Arg Pro Phe Ile Asp Glu Ala
                85                  90                  95

Lys Arg Leu Arg Ala Leu His Met Lys Glu His Pro Asp Tyr Lys Tyr
            100                 105                 110

Arg Pro Arg Arg Lys Thr Lys Thr Leu Met Lys Lys Asp Lys Tyr Thr
        115                 120                 125

Leu Pro Gly Gly Leu Leu Ala Pro Gly Gly Asn Ser Met Ala Ser Gly
    130                 135                 140

Val Gly Val Gly Ala Gly Leu Gly Ala Gly Val Asn Gln Arg Met Asp
145                 150                 155                 160

Ser Tyr Ala His Met Asn Gly Trp Ser Asn Gly Ser Tyr Ser Met Met
                165                 170                 175

Gln Glu Gln Leu Gly Tyr Pro Gln His Pro Gly Leu Asn Ala His Gly
            180                 185                 190

Ala Ala Gln Met Gln Pro Met His Arg Tyr Asp Val Ser Ala Leu Gln
        195                 200                 205

Tyr Asn Ser Met Thr Ser Ser Gln Thr Tyr Met Asn Gly Ser Pro Thr
    210                 215                 220

Tyr Ser Met Ser Tyr Ser Gln Gln Gly Thr Pro Gly Met Ala Leu Gly
225                 230                 235                 240

Ser Met Gly Ser Val Val Lys Ser Glu Ala Ser Ser Ser Pro Pro Val
                245                 250                 255

Val Thr Ser Ser Ser His Ser Arg Ala Pro Cys Gln Ala Gly Asp Leu
            260                 265                 270

Arg Asp Met Ile Ser Met Tyr Leu Pro Gly Ala Glu Val Pro Glu Pro
        275                 280                 285

Ala Ala Pro Ser Arg Leu His Met Ala Gln His Tyr Gln Ser Gly Pro
    290                 295                 300

Val Pro Gly Thr Ala Ile Asn Gly Thr Leu Pro Leu Ser His Met
305                 310                 315

<210>  7
<211>  954
<212>  DNA
<213>  homo sapiens

<400>  7
atgtacaaca tgatggagac ggagctgaag ccgccgggcc cgcagcaaac ttcggggggc      60

ggcggcggca actccaccgc ggcggcggcc ggcggcaacc agaaaaacag cccggaccgc     120

gtcaagcggc ccatgaatgc cttcatggtg tggtcccgcg ggcagcggcg caagatggcc     180

caggagaacc ccaagatgca caactcggag atcagcaagc gcctgggcgc cgagtggaaa     240

cttttgtcgg agacggagaa gcggccgttc atcgacgagg ctaagcggct gcgagcgctg     300

cacatgaagg agcacccgga ttataaatac cggccccggc ggaaaaccaa gacgctcatg     360

aagaaggata agtacacgct gcccggcggg ctgctggccc ccggcggcaa tagcatggcg     420

agcggggtcg gggtgggcgc cggcctgggc gcgggcgtga accagcgcat ggacagttac     480

gcgcacatga acggctggag caacggcagc tacagcatga tgcaggacca gctgggctac     540

ccgcagcacc cgggcctcaa tgcgcacggc gcagcgcaga tgcagcccat gcaccgctac     600

gacgtgagcg ccctgcagta caactccatg accagctcgc agacctacat gaacggctcg 660

cccacctaca gcatgtccta ctcgcagcag ggcacccctg gcatggctct tggctccatg 720

ggttcggtgg tcaagtccga ggccagctcc agcccccctg tggttacctc ttcctcccac 780

tccagggcgc cctgccaggc cggggacctc cgggacatga tcagcatgta tctccccggc 840

gccgaggtgc cggaacccgc cgcccccagc agacttcaca tgtcccagca ctaccagagc 900

ggcccggtgc ccggcacggc cattaacggc acactgcccc tctcacacat gtga 954

<210> 8
<211> 317
<212> PRT
<213> homo sapiens

<400> 8

<210>  9
<211>  918
<212>  DNA
<213>  mus musculus

<400>  9
atgagtgtgg gtcttcctgg tccccacagt ttgcctagtt ctgaggaagc atcgaattct      60

gggaacgcct catcaatgcc tgcagttttt catcccgaga actattcttg cttacaaggg     120

tctgctactg agatgctctg cacagaggct gcctctcctc gcccttcctc tgaagacctg     180

cctcttcaag gcagccctga ttcttctacc agtcccaaac aaaagctctc aagtcctgag     240

gctgacaagg gccctgagga ggaggagaac aaggtccttg ccaggaagca gaagatgcgg     300

actgtgttct ctcaggccca gctgtgtgca ctcaaggaca ggtttcagaa gcagaagtac     360

ctcagcctcc agcagatgca agaactctcc tccattctga acctgagcta taagcaggtt     420

aagacctggt ttcaaaacca aaggatgaag tgcaagcggt ggcagaaaaa ccagtggttg     480

aagactagca atggtctgat tcagaagggc tcagcaccag tggagtatcc cagcatccat     540

tgcagctatc cccagggcta tctggtgaac gcatctggaa gcctttccat gtggggcagc     600

cagacttgga ccaacccaac ttggagcagc cagacctgga ccaacccaac ttggaacaac     660

cagacctgga ccaacccaac ttggagcagc caggcctgga ccgctcagtc ctggaacggc 720

cagccttgga atgctgctcc gctccataac ttcggggagg actttctgca gccttacgta 780

cagttgcagc aaaacttctc tgccagtgat ttggaggtga atttggaagc cactagggaa 840

agccatgcgc attttagcac cccacaagcc ttggaattat tcctgaacta ctctgtgact 900

ccaccaggtg aaatatga 918

<210> 10
<211> 305
<212> PRT
<213> mus musculus

<400> 10

Met Ser Val Gly Leu Pro Gly Pro His Ser Leu Pro Ser Ser Glu Glu
1               5                   10                  15

Ala Ser Asn Ser Gly Asn Ala Ser Ser Met Pro Ala Val Phe His Pro
            20                  25                  30

Glu Asn Tyr Ser Cys Leu Gln Gly Ser Ala Thr Glu Met Leu Cys Thr
        35                  40                  45

Glu Ala Ala Ser Pro Arg Pro Ser Ser Glu Asp Leu Pro Leu Gln Gly
    50                  55                  60

Ser Pro Asp Ser Ser Thr Ser Pro Lys Gln Lys Leu Ser Ser Pro Glu
65                  70                  75                  80

Ala Asp Lys Gly Pro Glu Glu Glu Glu Asn Lys Val Leu Ala Arg Lys
                85                  90                  95

Gln Lys Met Arg Thr Val Phe Ser Gln Ala Gln Leu Cys Ala Leu Lys
            100                 105                 110

Asp Arg Phe Gln Lys Gln Lys Tyr Leu Ser Leu Gln Gln Met Gln Glu
        115                 120                 125

Leu Ser Ser Ile Leu Asn Leu Ser Tyr Lys Gln Val Lys Thr Trp Phe
    130                 135                 140

Gln Asn Gln Arg Met Lys Cys Lys Arg Trp Gln Lys Asn Gln Trp Leu
145                 150                 155                 160

Lys Thr Ser Asn Gly Leu Ile Gln Lys Gly Ser Ala Pro Val Glu Tyr
                165                 170                 175

Pro Ser Ile His Cys Ser Tyr Pro Gln Gly Tyr Leu Val Asn Ala Ser
            180                 185                 190

Gly Ser Leu Ser Met Trp Gly Ser Gln Thr Trp Thr Asn Pro Thr Trp
        195                 200                 205

Ser Ser Gln Thr Trp Thr Asn Pro Thr Trp Asn Asn Gln Thr Trp Thr
    210                 215                 220

Asn Pro Thr Trp Ser Ser Gln Ala Trp Thr Ala Gln Ser Trp Asn Gly
225                 230                 235                 240

Gln Pro Trp Asn Ala Ala Pro Leu His Asn Phe Gly Glu Asp Phe Leu
                245                 250                 255

Gln Pro Tyr Val Gln Leu Gln Gln Asn Phe Ser Ala Ser Asp Leu Glu
            260                 265                 270

Val Asn Leu Glu Ala Thr Arg Glu Ser His Ala His Phe Ser Thr Pro
        275                 280                 285

Gln Ala Leu Glu Leu Phe Leu Asn Tyr Ser Val Thr Pro Pro Gly Glu
    290                 295                 300

Ile
305

<210>  11
<211>  918
<212>  DNA
<213>  homo sapiens

<400>  11
atgagtgtgg atccagcttg tccccaaagc ttgccttgct ttgaagcatc cgactgtaaa      60

gaatcttcac ctatgcctgt gatttgtggg cctgaagaaa actatccatc cttgcaaatg     120

tcttctgctg agatgcctca cacggagact gtctctcctc ttccttcctc catggatctg     180

cttattcagg acagccctga ttcttccacc agtcccaaag gcaaacaacc cacttctgca     240

gagaagagtg tcgcaaaaaa ggaagacaag gtcccggtca agaaacagaa gaccagaact     300

gtgttctctt ccacccagct gtgtgtactc aatgatagat ttcagagaca gaaatacctc     360

agcctccagc agatgcaaga actctccaac atcctgaacc tcagctacaa acaggtgaag     420

acctggttcc agaaccagag aatgaaatct aagaggtggc agaaaaacaa ctggccgaag     480

aatagcaatg gtgtgacgca gaaggcctca gcacctacct accccagcct ttactcttcc     540

taccaccagg gatgcctggt gaacccgact gggaaccttc caatgtggag caaccagacc     600

tggaacaatt caacctggag caaccagacc cagaacatcc agtcctggag caaccactcc     660

tggaacactc agacctggtg cacccaatcc tggaacaatc aggcctggaa cagtcccttc     720

tataactgtg gagaggaatc tctgcagtcc tgcatgcagt tccagccaaa ttctcctgcc     780

agtgacttgg aggctgcctt ggaagctgct ggggaaggcc ttaatgtaat acagcagacc     840

actaggtatt ttagtactcc acaaaccatg gatttattcc taaactactc catgaacatg     900

caacctgaag acgtgtga                                                   918

<210>  12
<211>  305
<212>  PRT
<213>  homo sapiens

<400>  12

Met Ser Val Asp Pro Ala Cys Pro Gln Ser Leu Pro Cys Phe Glu Ala
1               5                   10                  15

Ser Asp Cys Lys Glu Ser Ser Pro Met Pro Val Ile Cys Gly Pro Glu
            20                  25                  30

Glu Asn Tyr Pro Ser Leu Gln Met Ser Ser Ala Glu Met Pro His Thr
        35                  40                  45

Glu Thr Val Ser Pro Leu Pro Ser Ser Met Asp Leu Leu Ile Gln Asp
    50                  55                  60

Ser Pro Asp Ser Ser Thr Ser Pro Lys Gly Lys Gln Pro Thr Ser Ala
65                  70                  75                  80

Glu Lys Ser Val Ala Lys Lys Glu Asp Lys Val Pro Val Lys Lys Gln
                85                  90                  95

Lys Thr Arg Thr Val Phe Ser Ser Thr Gln Leu Cys Val Leu Asn Asp
            100                 105                 110

Arg Phe Gln Arg Gln Lys Tyr Leu Ser Leu Gln Gln Met Gln Glu Leu
        115                 120                 125

Ser Asn Ile Leu Asn Leu Ser Tyr Lys Gln Val Lys Thr Trp Phe Gln
    130                 135                 140

Asn Gln Arg Met Lys Ser Lys Arg Trp Gln Lys Asn Asn Trp Pro Lys
145                 150                 155                 160

Asn Ser Asn Gly Val Thr Gln Lys Ala Ser Ala Pro Thr Tyr Pro Ser
                165                 170                 175

Leu Tyr Ser Ser Tyr His Gln Gly Cys Leu Val Asn Pro Thr Gly Asn
            180                 185                 190

Leu Pro Met Trp Ser Asn Gln Thr Trp Asn Asn Ser Thr Trp Ser Asn
        195                 200                 205

Gln Thr Gln Asn Ile Gln Ser Trp Ser Asn His Ser Trp Asn Thr Gln
    210                 215                 220

Thr Trp Cys Thr Gln Ser Trp Asn Asn Gln Ala Trp Asn Ser Pro Phe
225                 230                 235                 240

Tyr Asn Cys Gly Glu Glu Ser Leu Gln Ser Cys Met Gln Phe Gln Pro
                245                 250                 255

Asn Ser Pro Ala Ser Asp Leu Glu Ala Ala Leu Glu Ala Ala Gly Glu
            260                 265                 270

Gly Leu Asn Val Ile Gln Gln Thr Thr Arg Tyr Phe Ser Thr Pro Gln
        275                 280                 285

Thr Met Asp Leu Phe Leu Asn Tyr Ser Met Asn Met Gln Pro Glu Asp
    290                 295                 300

Val
305

<210>  13
<211>  1320
<212>  DNA
<213>  mus musculus

<400>  13
atgcccctca acgtgaactt caccaacagg aactatgacc tcgactacga ctccgtacag      60

ccctatttca tctgcgacga ggaagagaat ttctatcacc agcaacagca gagcgagctg     120

cagccgcccg cgcccagtga ggatatctgg aagaaattcg agctgcttcc caccccgccc     180

ctgtccccga gccgccgctc cgggctctgc tctccatcct atgttgcggt cgctacgtcc     240

ttctccccaa gggaagacga tgacggcggc ggtggcaact tctccaccgc cgatcagctg     300

gagatgatga ccgagttact tggaggagac atggtgaacc agagcttcat ctgcgatcct     360

gacgacgaga ccttcatcaa gaacatcatc atccaggact gtatgtggag cggtttctca     420

gccgctgcca agctggtctc ggagaagctg gcctcctacc aggctgcgcg caaagacagc     480

accagcctga gccccgcccg cgggcacagc gtctgctcca cctccagcct gtacctgcag     540

gacctcaccg ccgccgcgtc cgagtgcatt gacccctcag tggtctttcc ctacccgctc     600

aacgacagca gctcgcccaa atcctgtacc tcgtccgatt ccacggcctt ctctccttcc     660

tcggactcgc tgctgtcctc cgagtcctcc ccacgggcca gccctgagcc cctagtgctg     720

catgaggaga caccgcccac caccagcagc gactctgaag aagagcaaga agatgaggaa     780

gaaattgatg tggtgtctgt ggagaagagg caaacccctg ccaagaggtc ggagtcgggc 840
tcatctccat cccgaggcca cagcaaacct ccgcacagcc cactggtcct caagaggtgc 900
cacgtctcca ctcaccagca caactacgcc gcacccccct ccacaaggaa ggactatcca 960
gctgccaaga gggccaagtt ggacagtggc agggtcctga agcagatcag caacaaccgc 1020
aagtgctcca gccccaggtc ctcagacacg gaggaaaacg acaagaggcg gacacacaac 1080
gtcttggaac gtcagaggag gaacgagctg aagcgcagct tttttgccct gcgtgaccag 1140
atccctgaat tggaaaacaa cgaaaaggcc cccaaggtag tgatcctcaa aaaagccacc 1200
gcctacatcc tgtccattca agcagacgag cacaagctca cctctgaaaa ggacttattg 1260
aggaaacgac gagaacagtt gaaacacaaa ctcgaacagc ttcgaaactc tggtgcataa 1320

<210> 14
<211> 454
<212> PRT
<213> mus musculus

<400> 14

Leu Asp Phe Leu Trp Ala Leu Glu Thr Pro Gln Thr Ala Thr Thr Met
1               5                   10                  15

Pro Leu Asn Val Asn Phe Thr Asn Arg Asn Tyr Asp Leu Asp Tyr Asp
            20                  25                  30

Ser Val Gln Pro Tyr Phe Ile Cys Asp Glu Glu Glu Asn Phe Tyr His
        35                  40                  45

Gln Gln Gln Gln Ser Glu Leu Gln Pro Pro Ala Pro Ser Glu Asp Ile
    50                  55                  60

Trp Lys Lys Phe Glu Leu Leu Pro Thr Pro Pro Leu Ser Pro Ser Arg
65                  70                  75                  80

Arg Ser Gly Leu Cys Ser Pro Ser Tyr Val Ala Val Ala Thr Ser Phe
                85                  90                  95

Ser Pro Arg Glu Asp Asp Asp Gly Gly Gly Gly Asn Phe Ser Thr Ala
            100                 105                 110

Asp Gln Leu Glu Met Met Thr Glu Leu Leu Gly Gly Asp Met Val Asn
        115                 120                 125

Gln Ser Phe Ile Cys Asp Pro Asp Asp Glu Thr Phe Ile Lys Asn Ile
    130                 135                 140

Ile Ile Gln Asp Cys Met Trp Ser Gly Phe Ser Ala Ala Ala Lys Leu
145                 150                 155                 160

Val Ser Glu Lys Leu Ala Ser Tyr Gln Ala Ala Arg Lys Asp Ser Thr
                165                 170                 175

Ser Leu Ser Pro Ala Arg Gly His Ser Val Cys Ser Thr Ser Ser Leu
            180                 185                 190

Tyr Leu Gln Asp Leu Thr Ala Ala Ala Ser Glu Cys Ile Asp Pro Ser
        195                 200                 205

Val Val Phe Pro Tyr Pro Leu Asn Asp Ser Ser Ser Pro Lys Ser Cys
    210                 215                 220

Thr Ser Ser Asp Ser Thr Ala Phe Ser Pro Ser Ser Asp Ser Leu Leu
225                 230                 235                 240

Ser Ser Glu Ser Ser Pro Arg Ala Ser Pro Glu Pro Leu Val Leu His
                245                 250                 255

Glu Glu Thr Pro Pro Thr Thr Ser Ser Asp Ser Glu Glu Glu Gln Glu
            260                 265                 270

Asp Glu Glu Glu Ile Asp Val Val Ser Val Glu Lys Arg Gln Thr Pro
        275                 280                 285

Ala Lys Arg Ser Glu Ser Gly Ser Ser Pro Ser Arg Gly His Ser Lys
    290                 295                 300

Pro Pro His Ser Pro Leu Val Leu Lys Arg Cys His Val Ser Thr His
305                 310                 315                 320

Gln His Asn Tyr Ala Ala Pro Pro Ser Thr Arg Lys Asp Tyr Pro Ala
                325                 330                 335

Ala Lys Arg Ala Lys Leu Asp Ser Gly Arg Val Leu Lys Gln Ile Ser
            340                 345                 350

Asn Asn Arg Lys Cys Ser Ser Pro Arg Ser Ser Asp Thr Glu Glu Asn
        355                 360                 365

Asp Lys Arg Arg Thr His Asn Val Leu Glu Arg Gln Arg Arg Asn Glu
    370                 375                 380

Leu Lys Arg Ser Phe Phe Ala Leu Arg Asp Gln Ile Pro Glu Leu Glu
385                 390                 395                 400

Asn Asn Glu Lys Ala Pro Lys Val Val Ile Leu Lys Lys Ala Thr Ala
                405                 410                 415

Tyr Ile Leu Ser Ile Gln Ala Asp Glu His Lys Leu Thr Ser Glu Lys
            420                 425                 430

Asp Leu Leu Arg Lys Arg Arg Glu Gln Leu Lys His Lys Leu Glu Gln
        435                 440                 445

Leu Arg Asn Ser Gly Ala
    450

<210>  15
<211>  1320
<212>  DNA
<213>  homo sapiens

<400>  15
atgcccctca acgttagctt caccaacagg aactatgacc tcgactacga ctcggtgcag      60

ccgtatttct actgcgacga ggaggagaac ttctaccagc agcagcagca gagcgagctg     120

cagcccccgg cgcccagcga ggatatctgg aagaaattcg agctgctgcc caccccgccc     180

ctgtccccta gccgccgctc cgggctctgc tcgccctcct acgttgcggt cacacccttc     240

tcccttcggg gagacaacga cggcggtggc gggagcttct ccacggccga ccagctggag     300

atggtgaccg agctgctggg aggagacatg gtgaaccaga gtttcatctg cgacccggac     360

gacgagacct tcatcaaaaa catcatcatc caggactgta tgtggagcgg cttctcggcc     420

gccgccaagc tcgtctcaga gaagctggcc tcctaccagg ctgcgcgcaa agacagcggc     480

agcccgaacc ccgcccgcgg ccacagcgtc tgctccacct ccagcttgta cctgcaggat     540

ctgagcgccg ccgcctcaga gtgcatcgac ccctcggtgg tcttccccta ccctctcaac     600

gacagcagct cgcccaagtc ctgcgcctcg caagactcca gcgccttctc tccgtcctcg     660

gattctctgc tctcctcgac ggagtcctcc ccgcagggca gccccgagcc cctggtgctc     720

catgaggaga caccgcccac caccagcagc gactctgagg aggaacaaga agatgaggaa     780

gaaatcgatg ttgtttctgt ggaaaagagg caggctcctg gcaaaaggtc agagtctgga     840

tcaccttctg ctggaggcca cagcaaacct cctcacagcc cactggtcct caagaggtgc     900

cacgtctcca cacatcagca caactacgca gcgcctccct ccactcggaa ggactatcct     960

gctgccaaga gggtcaagtt ggacagtgtc agagtcctga gacagatcag caacaaccga    1020

aaatgcacca gccccaggtc ctcggacacc gaggagaatg tcaagaggcg aacacacaac    1080

gtcttggagc gccagaggag gaacgagcta aaacggagct tttttgccct gcgtgaccag    1140

atcccggagt tggaaaacaa tgaaaaggcc cccaaggtag ttatccttaa aaaagccaca    1200

gcatacatcc tgtccgtcca agcagaggag caaaagctca tttctgaaga ggacttgttg    1260

cggaaacgac gagaacagtt gaaacacaaa cttgaacagc tacggaactc ttgtgcgtaa    1320

<210>  16
<211>  454
<212>  PRT
<213>  homo sapiens

<400>  16

Leu Asp Phe Phe Arg Val Val Glu Asn Gln Gln Pro Pro Ala Thr Met
1               5                   10                  15

Pro Leu Asn Val Ser Phe Thr Asn Arg Asn Tyr Asp Leu Asp Tyr Asp
            20                  25                  30

Ser Val Gln Pro Tyr Phe Tyr Cys Asp Glu Glu Glu Asn Phe Tyr Gln
        35                  40                  45

Gln Gln Gln Gln Ser Glu Leu Gln Pro Pro Ala Pro Ser Glu Asp Ile
    50                  55                  60

Trp Lys Lys Phe Glu Leu Leu Pro Thr Pro Pro Leu Ser Pro Ser Arg
65                  70                  75                  80

Arg Ser Gly Leu Cys Ser Pro Ser Tyr Val Ala Val Thr Pro Phe Ser
                85                  90                  95

Leu Arg Gly Asp Asn Asp Gly Gly Gly Gly Ser Phe Ser Thr Ala Asp
            100                 105                 110

Gln Leu Glu Met Val Thr Glu Leu Leu Gly Gly Asp Met Val Asn Gln
        115                 120                 125

Ser Phe Ile Cys Asp Pro Asp Asp Glu Thr Phe Ile Lys Asn Ile Ile
    130                 135                 140

Ile Gln Asp Cys Met Trp Ser Gly Phe Ser Ala Ala Ala Lys Leu Val
145                 150                 155                 160

Ser Glu Lys Leu Ala Ser Tyr Gln Ala Ala Arg Lys Asp Ser Gly Ser
                165                 170                 175

Pro Asn Pro Ala Arg Gly His Ser Val Cys Ser Thr Ser Ser Leu Tyr
            180                 185                 190

Leu Gln Asp Leu Ser Ala Ala Ala Ser Glu Cys Ile Asp Pro Ser Val
        195                 200                 205

Val Phe Pro Tyr Pro Leu Asn Asp Ser Ser Ser Pro Lys Ser Cys Ala
    210                 215                 220

Ser Gln Asp Ser Ser Ala Phe Ser Pro Ser Ser Asp Ser Leu Leu Ser
225                 230                 235                 240

Ser Thr Glu Ser Ser Pro Gln Gly Ser Pro Glu Pro Leu Val Leu His
                245                 250                 255

Glu Glu Thr Pro Pro Thr Thr Ser Ser Asp Ser Glu Glu Glu Gln Glu
            260                 265                 270

Asp Glu Glu Glu Ile Asp Val Val Ser Val Glu Lys Arg Gln Ala Pro
        275                 280                 285

Gly Lys Arg Ser Glu Ser Gly Ser Pro Ser Ala Gly Gly His Ser Lys
    290                 295                 300

Pro Pro His Ser Pro Leu Val Leu Lys Arg Cys His Val Ser Thr His
305                 310                 315                 320

Gln His Asn Tyr Ala Ala Pro Pro Ser Thr Arg Lys Asp Tyr Pro Ala
                325                 330                 335

Ala Lys Arg Val Lys Leu Asp Ser Val Arg Val Leu Arg Gln Ile Ser
            340                 345                 350

Asn Asn Arg Lys Cys Thr Ser Pro Arg Ser Ser Asp Thr Glu Glu Asn
        355                 360                 365

Val Lys Arg Arg Thr His Asn Val Leu Glu Arg Gln Arg Arg Asn Glu
    370                 375                 380

Leu Lys Arg Ser Phe Phe Ala Leu Arg Asp Gln Ile Pro Glu Leu Glu
385                 390                 395                 400

Asn Asn Glu Lys Ala Pro Lys Val Val Ile Leu Lys Lys Ala Thr Ala
                405                 410                 415

Tyr Ile Leu Ser Val Gln Ala Glu Glu Gln Lys Leu Ile Ser Glu Glu
            420                 425                 430

Asp Leu Leu Arg Lys Arg Arg Glu Gln Leu Lys His Lys Leu Glu Gln
        435                 440                 445

Leu Arg Asn Ser Cys Ala
    450

<210>  17
<211>  1425
<212>  DNA
<213>  mus musculus

<400>  17
atggctgtca gcgacgctct gctcccgtcc ttctccacgt tcgcgtccgg cccggcggga      60

agggagaaga cactgcgtcc agcaggtgcc ccgactaacc gttggcgtga ggaactctct     120

cacatgaagc gacttccccc acttcccggc cgcccctacg acctggcggc gacggtggcc     180

acagacctgg agagtggcgg agctggtgca gcttgcagca gtaacaaccc ggccctccta     240

gcccggaggg agaccgagga gttcaacgac ctcctggacc tagactttat cctttccaac     300

tcgctaaccc accaggaatc ggtggccgcc accgtgacca cctcggcgtc agcttcatcc     360

tcgtcttccc cggcgagcag cggccctgcc agcgcgccct ccacctgcag cttcagctat     420

ccgatccggg ccgggggtga cccgggcgtg gctgccagaa acacaggtgg agggctcctc     480

tacagccgag aatctgcgcc acctcccacg gcccccttca acctggggga catcaatgac     540

gtgagcccct cgggcggctt cgtggctgag ctcctgcggc cggagttgga cccagtatac     600

attccgccac agcagcctca gccgccaggt gggggctgga tgggcaagtt tgtgctgaag     660

gcgtctctga ccacccctgg cagcgagtac agcagccctt cggtcatcag tgttagcaaa     720

ggaagcccag acggcagcca ccccgtggta gtggcgccct acagcggtgg cccgccgcgc     780

atgtgcccca agattaagca agaggcggtc ccgtcctgca cggtcagccg gtccctagag     840

gcccatttga gcgctggacc ccagctcagc aacggccacc ggcccaacac acacgacttc     900

cccctggggc ggcagctccc caccaggact acccctacac tgagtcccga ggaactgctg     960

aacagcaggg actgtcaccc tggcctgcct cttcccccag gattccatcc ccatccgggg    1020

gccaactacc ctcctttcct gccagaccag atgcagtcac aagtcccctc tctccattat    1080

caagagctca tgccaccggg ttcctgcctg ccagaggagc ccaagccaaa gaggggaaga    1140

aggtcgtggc cccggaaaag aacagccacc cacacttgtg actatgcagg ctgtggcaaa    1200

acctatacca agagttctca tctcaaggca cacctgcgaa ctcacacagg cgagaaacct    1260

taccactgtg actgggacgg ctgtgggtgg aaattcgccc gctccgatga actgaccagg    1320

cactaccgca aacacacagg gcaccggccc tttcagtgcc agaagtgtga cagggccttt    1380

tccaggtcgg accaccttgc cttacacatg aagaggcact tttaa                    1425

<210>  18
<211>  474
<212>  PRT
<213>  mus musculus

<400>  18

Met Ala Val Ser Asp Ala Leu Leu Pro Ser Phe Ser Thr Phe Ala Ser
1               5                   10                  15

Gly Pro Ala Gly Arg Glu Lys Thr Leu Arg Pro Ala Gly Ala Pro Thr
            20                  25                  30

Asn Arg Trp Arg Glu Glu Leu Ser His Met Lys Arg Leu Pro Pro Leu
        35                  40                  45

Pro Gly Arg Pro Tyr Asp Leu Ala Ala Thr Val Ala Thr Asp Leu Glu
    50                  55                  60

Ser Gly Gly Ala Gly Ala Ala Cys Ser Ser Asn Asn Pro Ala Leu Leu
65                  70                  75                  80

Ala Arg Arg Glu Thr Glu Glu Phe Asn Asp Leu Leu Asp Leu Asp Phe
                85                  90                  95

Ile Leu Ser Asn Ser Leu Thr His Gln Glu Ser Val Ala Ala Thr Val
            100                 105                 110

Thr Thr Ser Ala Ser Ala Ser Ser Ser Ser Ser Pro Ala Ser Ser Gly
        115                 120                 125

Pro Ala Ser Ala Pro Ser Thr Cys Ser Phe Ser Tyr Pro Ile Arg Ala
    130                 135                 140

Gly Gly Asp Pro Gly Val Ala Ala Arg Asn Thr Gly Gly Gly Leu Leu
145                 150                 155                 160

Tyr Ser Arg Glu Ser Ala Pro Pro Pro Thr Ala Pro Phe Asn Leu Gly
                165                 170                 175

Asp Ile Asn Asp Val Ser Pro Ser Gly Gly Phe Val Ala Glu Leu Leu
            180                 185                 190

Arg Pro Glu Leu Asp Pro Val Tyr Ile Pro Pro Gln Gln Pro Gln Pro
        195                 200                 205

Pro Gly Gly Gly Leu Met Gly Lys Phe Val Leu Lys Ala Ser Leu Thr
    210                 215                 220

Thr Pro Gly Ser Glu Tyr Ser Ser Pro Ser Val Ile Ser Val Ser Lys
225                 230                 235                 240

Gly Ser Pro Asp Gly Ser His Pro Val Val Val Ala Pro Tyr Ser Gly
                245                 250                 255

Gly Pro Pro Arg Met Cys Pro Lys Ile Lys Gln Glu Ala Val Pro Ser
            260                 265                 270

Cys Thr Val Ser Arg Ser Leu Glu Ala His Leu Ser Ala Gly Pro Gln
        275                 280                 285

Leu Ser Asn Gly His Arg Pro Asn Thr His Asp Phe Pro Leu Gly Arg
    290                 295                 300

Gln Leu Pro Thr Arg Thr Thr Pro Thr Leu Ser Pro Glu Glu Leu Leu
305                 310                 315                 320

Asn Ser Arg Asp Cys His Pro Gly Leu Pro Leu Pro Pro Gly Phe His
                325                 330                 335

Pro His Pro Gly Ala Asn Tyr Pro Pro Phe Leu Pro Asp Gln Met Gln
            340                 345                 350

Ser Gln Val Pro Ser Leu His Tyr Gln Glu Leu Met Pro Pro Gly Ser
        355                 360                 365

Cys Leu Pro Glu Glu Pro Lys Pro Lys Arg Gly Arg Arg Ser Trp Pro
    370                 375                 380

Arg Lys Arg Thr Ala Thr His Thr Cys Asp Tyr Ala Gly Cys Gly Lys
385                 390                 395                 400

Thr Tyr Thr Lys Ser Ser His Leu Lys Ala His Leu Arg Thr His Thr
                405                 410                 415

Gly Glu Lys Pro Tyr His Cys Asp Trp Asp Gly Cys Gly Trp Lys Phe
            420                 425                 430

Ala Arg Ser Asp Glu Leu Thr Arg His Tyr Arg Lys His Thr Gly His
        435                 440                 445

Arg Pro Phe Gln Cys Gln Lys Cys Asp Arg Ala Phe Ser Arg Ser Asp
    450                 455                 460

His Leu Ala Leu His Met Lys Arg His Phe
465                 470

<210>  19
<211>  1413
<212>  DNA
<213>  homo sapiens

<400>  19
atggctgtca gcgacgcgct gctcccatct ttctccacgt tcgcgtctgg cccggcggga      60

agggagaaga cactgcgtca agcaggtgcc ccgaataacc gctggcggga ggagctctcc     120

cacatgaagc gacttccccc agtgcttccc ggccgcccct atgacctggc ggcggcgacc     180

gtggccacag acctggagag cggcggagcc ggtgcggctt gcggcggtag caacctggcg     240

cccctacctc ggagagagac cgaggagttc aacgatctcc tggacctgga ctttattctc 300

tccaattcgc tgacccatcc tccggagtca gtggccgcca ccgtgtcctc gtcagcgtca 360

gcctcctctt cgtcgtcgcc gtcgagcagc ggccctgcca gcgcgccctc cacctgcagc 420

ttcacctatc cgatccgggc cgggaacgac ccgggcgtgg cgccgggcgg cacgggcgga 480

ggcctcctct atggcaggga gtccgctccc cctccgacgg ctcccttcaa cctggcggac 540

atcaacgacg tgagcccctc gggcggcttc gtggccgagc tcctgcggcc agaattggac 600

ccggtgtaca ttccgccgca gcagccgcag ccgccaggtg gcgggctgat gggcaagttc 660

gtgctgaagg cgtcgctgag cgcccctggc agcgagtacg gcagcccgtc ggtcatcagc 720

gtcagcaaag gcagccctga cggcagccac ccggtggtgg tggcgcccta caacggcggg 780

ccgccgcgca cgtgccccaa gatcaagcag gaggcggtct cttcgtgcac ccacttgggc 840

gctggacccc ctctcagcaa tggccaccgg ccggctgcac acgacttccc cctggggcgg 900

cagctcccca gcaggactac cccgaccctg ggtcttgagg aagtgctgag cagcagggac 960

tgtcaccctg ccctgccgct tcctcccggc ttccatcccc acccggggcc caattaccca 1020

tccttcctgc ccgatcagat gcagccgcaa gtcccgccgc tccattacca agagctcatg 1080

ccacccggtt cctgcatgcc agaggagccc aagccaaaga ggggaagacg atcgtggccc 1140

cggaaaagga ccgccaccca cacttgtgat tacgcgggct gcggcaaaac ctacacaaag 1200

agttcccatc tcaaggcaca cctgcgaacc cacacaggtg agaaacctta ccactgtgac 1260

tgggacggct gtggatggaa attcgcccgc tcagatgaac tgaccaggca ctaccgtaaa 1320

cacacggggc accgcccgtt ccagtgccaa aaatgcgacc gagcattttc caggtcggac 1380

cacctcgcct tacacatgaa gaggcatttt taa 1413

<210> 20
<211> 469
<212> PRT
<213> homo sapiens

<400> 20

Met Ala Val Ser Asp Ala Leu Leu Pro Ser Phe Ser Thr Phe Ala Ser
1               5                   10                  15

Gly Pro Ala Gly Arg Glu Lys Thr Leu Arg Gln Ala Gly Ala Pro Asn
            20                  25                  30

Asn Arg Trp Arg Glu Glu Leu Ser His Met Lys Arg Leu Pro Pro Val
        35                  40                  45

Leu Pro Gly Arg Pro Tyr Asp Leu Ala Ala Ala Thr Val Ala Thr Asp
    50                  55                  60

Leu Glu Ser Gly Gly Ala Gly Ala Ala Cys Gly Gly Ser Asn Leu Ala
65                  70                  75                  80

Pro Leu Pro Arg Arg Glu Thr Glu Glu Phe Asn Asp Leu Leu Asp Leu
                85                  90                  95

Asp Phe Ile Leu Ser Asn Ser Leu Thr His Pro Pro Glu Ser Val Ala
            100                 105                 110

Ala Thr Val Ser Ser Ser Ala Ser Ala Ser Ser Ser Ser Ser Pro Ser
        115                 120                 125

Ser Ser Gly Pro Ala Ser Ala Pro Ser Thr Cys Ser Phe Thr Tyr Pro
    130                 135                 140

Ile Arg Ala Gly Asn Asp Pro Gly Val Ala Pro Gly Gly Thr Gly Gly
145                 150                 155                 160

Gly Leu Leu Tyr Gly Arg Glu Ser Ala Pro Pro Pro Thr Ala Pro Phe
                165                 170                 175

Asn Leu Ala Asp Ile Asn Asp Val Ser Pro Ser Gly Gly Phe Val Ala
            180                 185                 190

Glu Leu Leu Arg Pro Glu Leu Asp Pro Val Tyr Ile Pro Pro Gln Gln
        195                 200                 205

Pro Gln Pro Pro Gly Gly Gly Leu Met Gly Lys Phe Val Leu Lys Ala
    210                 215                 220

Ser Leu Ser Ala Pro Gly Ser Glu Tyr Gly Ser Pro Ser Val Ile Ser
225                 230                 235                 240

Val Ser Lys Gly Ser Pro Asp Gly Ser His Pro Val Val Val Ala Pro
                245                 250                 255

Tyr Asn Gly Gly Pro Pro Arg Thr Cys Pro Lys Ile Lys Gln Glu Ala
            260                 265                 270

Val Ser Ser Cys Thr His Leu Gly Ala Gly Pro Pro Leu Ser Asn Gly
        275                 280                 285

His Arg Pro Ala Ala His Asp Phe Pro Leu Gly Arg Gln Leu Pro Ser
    290                 295                 300

Arg Thr Thr Pro Thr Leu Gly Leu Glu Glu Val Leu Ser Ser Arg Asp
305                 310                 315                 320

Cys His Pro Ala Leu Pro Leu Pro Pro Gly Phe His Pro His Pro Gly
                325                 330                 335

Pro Asn Tyr Pro Ser Phe Leu Pro Asp Gln Met Gln Pro Gln Val Pro
            340                 345                 350

Pro Leu His Tyr Gln Glu Leu Met Pro Pro Gly Ser Cys Met Pro Glu
        355                 360                 365

Glu Pro Lys Pro Lys Arg Gly Arg Arg Ser Trp Pro Arg Lys Arg Thr
    370                 375                 380

Ala Thr His Thr Cys Asp Tyr Ala Gly Cys Gly Lys Thr Tyr Thr Lys
385                 390                 395                 400

Ser Ser His Leu Lys Ala His Leu Arg Thr His Thr Gly Glu Lys Pro
                405                 410                 415

Tyr His Cys Asp Trp Asp Gly Cys Gly Trp Lys Phe Ala Arg Ser Asp
            420                 425                 430

Glu Leu Thr Arg His Tyr Arg Lys His Thr Gly His Arg Pro Phe Gln
        435                 440                 445

Cys Gln Lys Cys Asp Arg Ala Phe Ser Arg Ser Asp His Leu Ala Leu
    450                 455                 460

His Met Lys Arg His
465

<210>  21
<211>  630
<212>  DNA
<213>  mus musculus

<400>  21
   atgggctcgg tgtccaacca gcagtttgca ggtggctgcg ccaaggcagc ggagaaggcg      60
   ccagaggagg cgccgcctga cgcggcccga gcggcagacg agccgcagct gctgcacggg     120
   gccggcatct gtaagtggtt caacgtgcgc atggggttcg gcttcctgtc tatgaccgcc     180
   cgcgctgggg tcgcgctcga ccccccggtg gacgtctttg tgcaccagag caagctgcac     240
   atggaagggt tccgaagcct caaggagggt gaggcggtgg agttcacctt taagaagtct     300
   gccaagggtc tggaatccat ccgtgtcact ggccctggtg gtgtgttctg tattgggagt     360
   gagcggcggc caaaagggaa gaacatgcag aagcgaagat ccaaaggaga caggtgctac     420
   aactgcggtg ggctagacca tcatgccaag gaatgcaagc tgccacccca gcccaagaag     480
   tgccactttt gccaaagcat caaccatatg gtggcctcgt gtccactgaa ggcccagcag     540
   ggccccagtt ctcagggaaa gcctgcctac ttccgggagg aagaggaaga gatccacagc 600
   cctgccctgc tcccagaagc ccagaattga 630

<210> 22
<211> 209
<212> PRT
<213> mus musculus

<400> 22

Met Gly Ser Val Ser Asn Gln Gln Phe Ala Gly Gly Cys Ala Lys Ala
1               5                   10                  15

Ala Glu Lys Ala Pro Glu Glu Ala Pro Pro Asp Ala Ala Arg Ala Ala
            20                  25                  30

Asp Glu Pro Gln Leu Leu His Gly Ala Gly Ile Cys Lys Trp Phe Asn
        35                  40                  45

Val Arg Met Gly Phe Gly Phe Leu Ser Met Thr Ala Arg Ala Gly Val
    50                  55                  60

Ala Leu Asp Pro Pro Val Asp Val Phe Val His Gln Ser Lys Leu His
65                  70                  75                  80

Met Glu Gly Phe Arg Ser Leu Lys Glu Gly Glu Ala Val Glu Phe Thr
                85                  90                  95

Phe Lys Lys Ser Ala Lys Gly Leu Glu Ser Ile Arg Val Thr Gly Pro
            100                 105                 110

Gly Gly Val Phe Cys Ile Gly Ser Glu Arg Arg Pro Lys Gly Lys Asn
        115                 120                 125

Met Gln Lys Arg Arg Ser Lys Gly Asp Arg Cys Tyr Asn Cys Gly Gly
    130                 135                 140

Leu Asp His His Ala Lys Glu Cys Lys Leu Pro Pro Gln Pro Lys Lys
145                 150                 155                 160

Cys His Phe Cys Gln Ser Ile Asn His Met Val Ala Ser Cys Pro Leu
                165                 170                 175

Lys Ala Gln Gln Gly Pro Ser Ser Gln Gly Lys Pro Ala Tyr Phe Arg
            180                 185                 190

Glu Glu Glu Glu Glu Ile His Ser Pro Ala Leu Leu Pro Glu Ala Gln
        195                 200                 205

Asn

<210> 23
<211> 630
<212> DNA
<213> homo sapiens

<400> 23
   atgggctccg tgtccaacca gcagtttgca ggtggctgcg ccaaggcggc agaagaggcg 60
   cccgaggagg cgccggagga cgcggcccgg gcggcggacg agcctcagct gctgcacggt 120
   gcgggcatct gtaagtggtt caacgtgcgc atggggttcg gcttcctgtc catgaccgcc 180
   cgcgccgggg tcgcgctcga ccccccagtg gatgtctttg tgcaccagag taagctgcac 240
   atggaagggt tccggagctt gaaggagggt gaggcagtgg agttcacctt taagaagtca 300
   gccaagggtc tggaatccat ccgtgtcacc ggacctggtg gagtattctg tattgggagt 360
   gagaggcggc caaaaggaaa gagcatgcag aagcgcagat caaaaggaga caggtgctac 420
   aactgtggag gtctagatca tcatgccaag gaatgcaagc tgccacccca gcccaagaag 480
   tgccacttct gccagagcat cagccatatg gtagcctcat gtccgctgaa ggcccagcag 540
   ggccctagtg cacagggaaa gccaacctac tttcgagagg aagaagaaga aatccacagc 600
   cctaccctgc tcccggaggc acagaattga 630

<210> 24
<211> 209
<212> PRT
<213> homo sapiens

<400> 24

Met Gly Ser Val Ser Asn Gln Gln Phe Ala Gly Gly Cys Ala Lys Ala
1               5                   10                  15

Ala Glu Glu Ala Pro Glu Glu Ala Pro Glu Asp Ala Ala Arg Ala Ala
            20                  25                  30

Asp Glu Pro Gln Leu Leu His Gly Ala Gly Ile Cys Lys Trp Phe Asn
        35                  40                  45

Val Arg Met Gly Phe Gly Phe Leu Ser Met Thr Ala Arg Ala Gly Val
    50                  55                  60

Ala Leu Asp Pro Pro Val Asp Val Phe Val His Gln Ser Lys Leu His
65                  70                  75                  80

Met Glu Gly Phe Arg Ser Leu Lys Glu Gly Glu Ala Val Glu Phe Thr
                85                  90                  95

Phe Lys Lys Ser Ala Lys Gly Leu Glu Ser Ile Arg Val Thr Gly Pro
            100                 105                 110

Gly Gly Val Phe Cys Ile Gly Ser Glu Arg Arg Pro Lys Gly Lys Ser
        115                 120                 125

Met Gln Lys Arg Arg Ser Lys Gly Asp Arg Cys Tyr Asn Cys Gly Gly
    130                 135                 140

Leu Asp His His Ala Lys Glu Cys Lys Leu Pro Pro Gln Pro Lys Lys
145                 150                 155                 160

Cys His Phe Cys Gln Ser Ile Ser His Met Val Ala Ser Cys Pro Leu
                165                 170                 175

Lys Ala Gln Gln Gly Pro Ser Ala Gln Gly Lys Pro Thr Tyr Phe Arg
            180                 185                 190

Glu Glu Glu Glu Glu Ile His Ser Pro Thr Leu Leu Pro Glu Ala Gln
        195                 200                 205

Asn

<210> 25
<211> 34
<212> DNA
<213> Enterobacteria phage P1

<400> 25
ataacttcgt atagcataca ttatacgaag ttat 34